(19) Europäisches Patentamt / European Patent Office / Office européen des brevets

(11) EP 2 983 222 A1

(12) # EUROPEAN PATENT APPLICATION

published in accordance with Art. 153(4) EPC

(43) Date of publication:
**10.02.2016 Bulletin 2016/06**

(21) Application number: **14774301.7**

(22) Date of filing: **27.03.2014**

(51) Int Cl.:
***H01L 51/50*** *(2006.01)* ***C07D 403/14*** *(2006.01)*
***C07D 405/14*** *(2006.01)* ***C07D 413/04*** *(2006.01)*
***C07D 413/14*** *(2006.01)* ***C07D 471/14*** *(2006.01)*
***C07D 487/04*** *(2006.01)* ***C07D 487/14*** *(2006.01)*
***C07D 491/048*** *(2006.01)* ***C07D 491/147*** *(2006.01)*
***C07D 495/14*** *(2006.01)* ***C07D 498/14*** *(2006.01)*
***C07D 513/14*** *(2006.01)* ***C07D 513/16*** *(2006.01)*
***C09K 11/06*** *(2006.01)* ***G09F 9/30*** *(2006.01)*
***H01L 27/32*** *(2006.01)*

(86) International application number:
**PCT/JP2014/058808**

(87) International publication number:
**WO 2014/157494 (02.10.2014 Gazette 2014/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.03.2013 JP 2013071566**

(71) Applicant: **Konica Minolta, Inc.**
**Tokyo 100-7015 (JP)**

(72) Inventors:
- **SEKINE, Noboru**
  **Tokyo 100-7015 (JP)**
- **MIURA, Norio**
  **Tokyo 100-7015 (JP)**
- **INOUE, Satoru**
  **Tokyo 100-7015 (JP)**

(74) Representative: **Henkel, Breuer & Partner**
**Patentanwälte**
**Maximiliansplatz 21**
**80333 München (DE)**

(54) **MATERIAL FOR ORGANIC ELECTROLUMINESCENT ELEMENTS, ORGANIC ELECTROLUMINESCENT ELEMENT, DISPLAY DEVICE AND LIGHTING DEVICE**

(57)

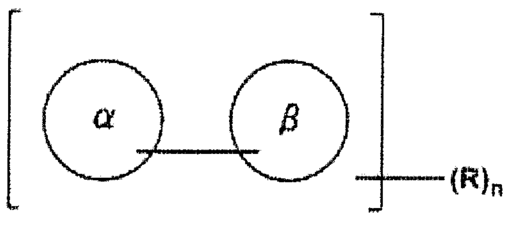

(1)

One objective of the present invention is to provide a material for organic electroluminescent elements, which has a high triplet excitation energy. Another objective of the present invention is to provide: an organic electroluminescent element which uses the material for organic electroluminescent elements and has high luminous efficiency and excellent durability; a lighting device; and a display device. A material for organic electroluminescent elements according to the present invention is characterized by having a structure represented by general formula (1). (In general formula (1), ring $\alpha$ and ring $\beta$ respectively represent a five-membered aromatic heterocyclic group having a specific structure, and are bonded with each other at arbitrary positions; R represents a hydrogen atom or a substituent that is substituted at an arbitrary position of ring $\alpha$ and/or ring $\beta$; and n represents an integer of 1-8.) AA General formula (1)

EP 2 983 222 A1

## Description

Technical Field

The present invention relates to a material for organic electroluminescent elements, and an organic electroluminescent element, a display device and a lighting device each using the material, and, to be more specific, relates to a material for organic electroluminescent elements having high luminous efficiency and durability, and an organic electroluminescent element, a display device and a lighting device each using the material.

Background Art

An organic electroluminescent element (hereinafter also called an "organic EL element") is a thin-film type completely-solid state element having an organic functional layer (a single-layer part or a multilayer part) containing an organic luminescent substance between an anode and a cathode. When a voltage is applied to the organic EL element, electrons and holes are injected from the cathode and the anode, respectively. An organic EL element is one which makes use of light discharged by excitons when the excitons transfer from an excited state to radiative decay, the excitons being produced through recombination of the electrons and holes in the light emitting layer (organic luminescent substance-containing layer) . This is a technique expected to be used for flat displays and lighting devices of the next generation.

There has been reported by Princeton University an organic EL element making use of phosphorescence emitted from an excited triplet state. This organic EL element can achieve luminous efficiency four times higher than an organic EL element which makes use of fluorescence emission. Starting from development of a phosphorescent material, researches and developments about the layer structure and electrodes have been conducted all over the world.

As described above, the phosphorescence emission system is a system having very high potential but greatly different from the fluorescence emission system, and how to control the position of luminescent center, in particular, how to carry out the recombination in a light emitting layer and stable light emission, has been an important technical object to increase efficiency and life of the element.

Then, in recent years, development of a multilayer element provided with not only a light emitting layer but also a hole transport layer located on the anode side of the light emitting layer, an electron transport layer located on the cathode side thereof and the like has been actively conducted. Further, a light emitting layer often employs a mixed layer using a phosphorescent compound as a luminescent dopant and a host compound.

Meanwhile, from the point of materials, creation of a new material to increase element performance has been highly expected. In particular, to make use of blue phosphoresce emission, because a blue phosphorescent compound itself has high triplet excitation energy ($T_1$), development of a peripheral material having triplet excitation energy sufficiently higher than the blue phosphorescent compound has been strongly demanded, and materials for organic EL elements using imidazole and the like have also been reported. (Refer to, for example, Patent Documents 1 and 2.)

However, even when the techniques described in Patent Documents 1 and 2 are used, there are still some problems in luminous efficiency and durability from the point of practical performance, and therefore development of a material exhibiting higher efficiency and higher durability has been demanded.

Related Art Documents

Patent Documents

Patent Document 1: Japanese Patent Application Publication No. 2007-243101
Patent Document 2: International Patent Application Publication No. 2012/051667

Summary of the Invention

Problems to be Solved by the Invention

The present invention has been conceived in view of the above problems and circumstances, and an object thereof is to provide a material for organic electroluminescent elements having high triplet excitation energy. Other objects thereof are to provide an organic electroluminescent element, a lighting device and a display device each using the material for organic electroluminescent elements, thereby having high luminous efficiency and excellent durability.

Means for Solving the Problems

The present inventors have examined the causes and the like of the above problems in order to achieve the above objects and found out that an organic compound having a specific structure is effective in achieving the above objects, thereby reaching the present invention.

That is, the above objects of the present invention are achieved by the following means.

1. A material for organic electroluminescent elements including a structure represented by the following General Formula (1).

[Chem. 1]

General Formula (1)

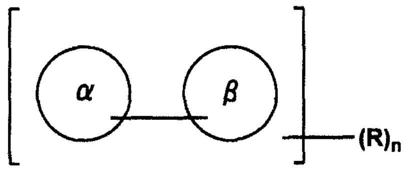

In the General Formula (1), a ring α and a ring β respectively represent aromatic heterocyclic groups each derived from pyrrole, furan, thiophene, pyrazole, 1,2,3-triazole, 1,2,4-triazole, tetrazole, oxazole, isoxazole, oxadiazole, thiazole, isothiazole or thiadiazole, and are linked with each other through arbitrary positions; R represents a hydrogen atom or a substituent substituted at an arbitrary position of at least one of the ring α and the ring β; and n represents an integer of 1 to 8.

2. The material for organic electroluminescent elements according to the item 1, wherein the structure represented by the General Formula (1) is a structure represented by the following General Formula (2).

[Chem. 2]

General Formula (2)

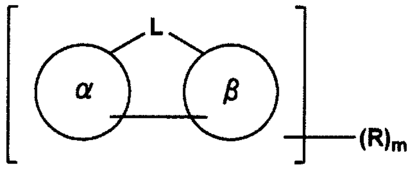

In the General Formula (2), a ring α, a ring β and R are synonymous with the ring α, ring β and R in the General Formula (1), respectively; m represents an integer of 1 to 6; and L represents a divalent linking group.

3. The material for organic electroluminescent elements according to the item 1, wherein the structure represented by the General Formula (1) is a structure represented by the following General Formula (1-1).

[Chem. 3]

General Formula (1-1)

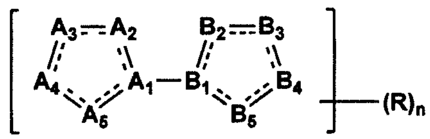

In the General Formula (1-1), $A_1$, $A_2$, $A_3$, $A_4$, $A_5$, $B_1$, $B_2$, $B_3$, $B_4$ and $B_5$ each represent a carbon atom, a nitrogen atom, an oxygen atom or a sulfur atom, and $A_1$ to $A_5$ and $B_1$ to $B_5$ respectively form 5-membered aromatic heterocycles each derived from pyrrole, furan, thiophene, pyrazole, 1,2,3-triazole, 1,2,4-triazole, tetrazole, oxazole, isoxazole, oxadiazole, thiazole, isothiazole or thiadiazole; R represents a hydrogen atom or a substituent substituted at an arbitrary position of at least one of the two aromatic heterocyclic groups; and n represents an integer of 1 to 8.

4. The material for organic electroluminescent elements according to the item 2, wherein the structure represented by the General Formula (2) is a structure represented by the following General Formula (2-1).

[Chem. 4]

General Formula (2-1)

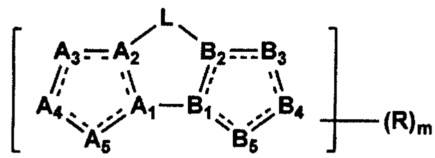

In the General Formula (2-1), $A_1$, $A_2$, $A_3$, $A_4$, $A_5$, $B_1$, $B_2$, $B_3$, $B_4$ and $B_5$ each represent a carbon atom, a nitrogen atom, an oxygen atom or a sulfur atom, and $A_1$ to $A_5$ and $B_1$ to $B_5$ respectively form 5-membered aromatic heterocycles each derived from pyrrole, furan, thiophene, pyrazole, 1,2,3-triazole, 1,2,4-triazole, tetrazole, oxazole, isoxazole, thiazole or isothiazole; R represents a hydrogen atom or a substituent substituted at an arbitrary position of at least one of the two aromatic heterocyclic groups; m represents an integer of 1 to 8; and L represents a divalent linking group.

5. An organic electroluminescent element including an organic layer including at least a light emitting layer interposed between an anode and a cathode, wherein any of the organic layer contains the material for organic electroluminescent elements according to any one of the items 1 to 4.

6. The organic electroluminescent element according to the item 5, wherein the light emitting layer contains a phosphorescent compound.

7. The organic electroluminescent element according to the item 6, wherein the phosphorescent compound has a structure represented by the following General Formula (DP).

[Chem. 5]

General Formula (DP)

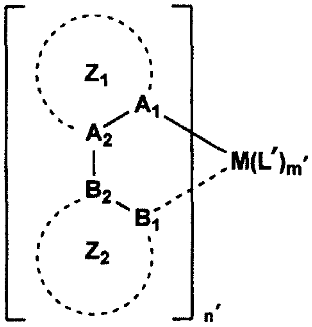

In the Formula, M represents Ir, Pt, Rh, Ru, Ag, Cu or Os; $A_1$, $A_2$, $B_1$ and $B_2$ each represent a carbon atom or a nitrogen atom; a ring $Z_1$ represents a 6-membered aromatic hydrocarbon ring or a 5- or 6-membered aromatic heterocycle formed with $A_1$ and $A_2$; a ring $Z_2$ represents a 5- or 6-membered aromatic heterocycle formed with $B_1$ and $B_2$; the ring $Z_1$ and the ring $Z_2$ may respectively have substituents, the substituents may be bonded with each other to form a condensed ring structure, and the substituents of ligands may be bonded with each other so that the ligands are linked with each other; L' represents a monoanionic bidentate ligand coordinated to M; m' represents an integer of 0 to 2, and n' represents an integer of 1 to 3, provided that m' + n' is 2 or 3; and when n' is 2 or more, the ligands represented by the ring $Z_1$ and the ring $Z_2$ may be the same or different from each other, and when m' is 2 or more, L's may be the same or different from each other.

8. A display device including the organic electroluminescent element according to any one of the items 5 to 7.

9. A lighting device including the organic electroluminescent element according to any one of the items 5 to 7.

Advantageous Effects of the Invention

According to the means of the present invention, a material for organic electroluminescent elements having high triplet excitation energy can be provided. Further, an organic electroluminescent element, a lighting device and a display device each using the material for organic electroluminescent elements, thereby having high luminous efficiency and excellent durability, can be provided.

Although appearance mechanism of the effects of the present invention and action mechanism thereof are not clear yet, they are conjectured as follows.

That is, the material for organic EL elements of the present invention is characterized in that a ring $\alpha$ and a ring $\beta$ are both 5-membered aromatic heterocycles each derived from pyrrole, furan, thiophene, pyrazole, 1,2,3-triazole, 1,2,4-triazole, tetrazole, oxazole, isoxazole, oxadiazole, thiazole, isothiazole or thiadiazole, and the ring $\alpha$ and the ring $\beta$ are linked with each other through arbitrary positions.

It is conjectured that by having this structure, the material for organic EL elements can have high triplet excitation energy, and carrier transfer in an organic EL element becomes adjustable by adjustment of HOMO level and LUMO level, whereby both high luminous efficiency and durability can be achieved. The details are described below.

Brief Description of the Drawings

FIG. 1 is a schematic view showing an example of a display device constituted of organic EL elements.

FIG. 2 is a schematic view of a display section of the display device shown in FIG. 1.

FIG. 3 is a circuit diagram of a pixel of the display device shown in FIG. 1.

FIG. 4 is a schematic view of a full-color display device employing a passive matrix system.

FIG. 5 is a schematic view of a lighting device.

FIG. 6 is a cross-sectional view of the lighting device.

FIG. 7A is a schematic configuration view of a full-color organic EL display device.

FIG. 7B is a schematic configuration view of the full-color organic EL display device.

FIG. 7C is a schematic configuration view of the full-color organic EL display device.
FIG. 7D is a schematic configuration view of the full-color organic EL display device.
FIG. 7E is a schematic configuration view of the full-color organic EL display device.

Embodiments for Carrying out the Invention

A material for organic electroluminescent elements of the present invention has a structure represented by the above General Formula (1) . This feature is a technical feature common to the inventions of claims 1 to 9.

As an embodiment of the present invention, the structure represented by the above General Formula (1) is preferably a structure represented by the above General Formula (2).

The structure represented by the above General Formula (1) is preferably a structure represented by the above General Formula (1-1), and the structure represented by the above General Formula (2) is preferably a structure represented by the above General Formula (2-1).

An organic electroluminescent element of the present invention is an organic electroluminescent element including an organic layer(s) including at least a light emitting layer interposed between an anode and a cathode, wherein any of the organic layer(s) contains the material for organic electroluminescent elements. Preferably, in the organic electroluminescent element, the light emitting layer contains a phosphorescent compound. Preferably, the phosphorescent compound has a structure represented by the above General Formula (DP).

The organic electroluminescent element of the present invention is preferably used in a display device or a lighting device.

Hereinafter, the present invention, its constituents and embodiments for carrying out the present invention are detailed.

Note that, in the present invention, "- (to)" between values is used to mean that the values before and after the sign are inclusive as the lower limit and the upper limit.

<<Compound Represented by General Formula (1)>>

A material for organic electroluminescent elements of the present invention has a structure represented by the General Formula (1).

[Chem. 6]

General Formula (1)

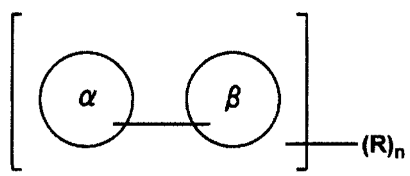

In the General Formula (1), a ring α and a ring β respectively represent aromatic heterocyclic groups each derived from pyrrole, furan, thiophene, pyrazole, 1,2,3-triazole, 1,2,4-triazole, tetrazole, oxazole, isoxazole, oxadiazole, thiazole, isothiazole or thiadiazole, and are linked with each other through arbitrary positions; R represents a hydrogen atom or a substituent substituted at an arbitrary position of at least one of the ring α and the ring β; and n represents an integer of 1 to 8.

The material for organic EL elements of the present invention is characterized in that the ring α and the ring β are both 5-membered aromatic heterocyclic groups, and the ring α and the ring β are linked with each other by a single bond.

By having this structure, the material for organic EL elements can have high triplet excitation energy, and carrier transfer in an organic EL element becomes adjustable through adjustment of HOMO level and LUMO level, whereby both high luminous efficiency and durability can be achieved.

Although the 5-membered aromatic heterocyclic group has been examined centering on imidazole, it still has problems, for example, in heat resistance and moist-heat resistance as a compound and also in durability such as an element life when used in an organic EL element.

In the 5-membered aromatic heterocyclic group of the present invention, pyrrole, furan, thiophene, pyrazole,

1,2,3-triazole, 1,2,4-triazole, tetrazole, oxazole, isoxazole, oxadiazole, thiazole, isothiazole or thiadiazole is used for the material for organic EL elements. This is assumed to be a reason why an organic EL element having high durability can be provided.

The causes of the problems which arise when imidazole is used are still unclear, but it is assumed that maldistribution of charges on constituent atoms of the imidazole ring, pKa and the like have some relationship with the causes of deterioration which occurs in an organic EL element.

The material for organic EL elements of the present invention has a structure of two aromatic heterocyclic groups linked, which increases stability and safety as a compound as compared with a 5-membered monocyclic aromatic heterocycle(s), and it is assumed that this increase in stability of the compound itself contributes to increase in durability of an organic EL element.

R represents a hydrogen atom or a substituent substituted at an arbitrary position of the ring $\alpha$ and/or the ring $\beta$. Examples of the substituent include an alkyl group, an alkenyl group, an alkoxy group, an alkynyl group, a carbonyl group, an amino group, a silyl group, a phosphine oxide group, an arylalkyl group, an aryl group, a heteroaryl group, a nonaromatic hydrocarbon ring group and a nonaromatic heterocyclic group. These substituents may further have a substituent. The substituent (s) is preferably an aryl group, a heteroaryl group, a silyl group or an alkyl group, far preferably a phenyl group, a carbazolyl group, a dibenzofuranyl group, a dibenzothiophenyl group, a triphenylsilyl group, a methyl group or an isopropyl group, and still far preferably a phenyl group, a carbazolyl group, a dibenzofuranyl group, a dibenzothiophenyl group or a triphenylsilyl group. These substituents may further have a substituent. Further, the substituents may be linked with each other to form a ring.

n represents an integer of 1 to 8. n is preferably 1 to 6, far preferably 1 to 4, and still far preferably 1 to 3.

A combination of the ring $\alpha$ and the ring $\beta$ is not particularly limited, but preferably at least one of them is a nitrogen-containing aromatic ring, and far preferably both of them are nitrogen-containing automatic rings.

Each of the ring $\alpha$ and the ring $\beta$ may have a condensed ring structure formed of substituents linked with each other. The formed condensed ring structure may be a saturated ring, an unsaturated ring or an aromatic ring, but preferably a saturated ring or an aromatic ring.

The ring $\alpha$ and the ring $\beta$ may be monocyclic or have condensed ring structures, but preferably one of them is monocyclic, and far preferably both of them are monocyclic.

The structure represented by the above General Formula (1) can be a structure represented by the following General Formula (1-1).

[Chem. 7]

General Formula (1-1)

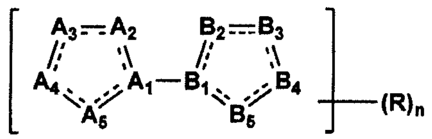

In the General Formula (1-1), $A_1$, $A_2$, $A_3$, $A_4$, $A_5$, $B_1$, $B_2$, $B_3$, $B_4$ and $B_5$ each represent a carbon atom, a nitrogen atom, an oxygen atom or a sulfur atom, and $A_1$ to $A_5$ and $B_1$ to $B_5$ respectively form 5-membered heterocycles below; R represents a hydrogen atom or a substituent substituted at an arbitrary position of at least one of the two aromatic heterocyclic groups; and n represents an integer of 1 to 8.

As stated in the General Formula (1), each 5-membered heterocycle is pyrrole, furan, thiophene, pyrazole, 1,2,3-triazole, 1,2,4-triazole, tetrazole, oxazole, isoxazole, oxadiazole, thiazole, isothiazole or thiadiazole.

A doublet of a solid line and a broken line represents a single bond or a double bond, and the ring $\alpha$ formed of $A_1$ to $A_5$ and the ring $\beta$ formed of $B_1$ to $B_5$ are both aromatic rings. The other symbols are synonymous with those in the General Formula (1).

Further, the structure represented by the General Formula (1-1) can be preferably a structure represented by the following General Formula (1-2) or General Formula (1-3).

[Chem. 8]

General Formula (1-2) General Formula (1-3)

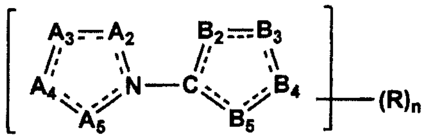

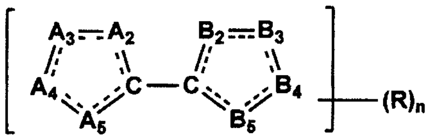

The symbols in the General Formulae (1-2) and (1-3) are synonymous with those in the General Formula (1-1). Further, the General Formula (1) can be represented by any of the following General Formula (1-A) to General Formula (1-I).

[Chem. 9]

General Formula (1-A)

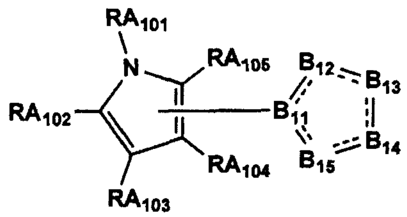

General Formula (1-B)

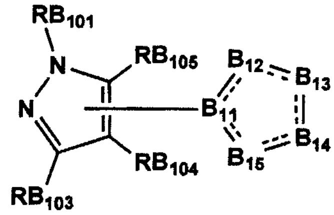

General Formula (1-C)

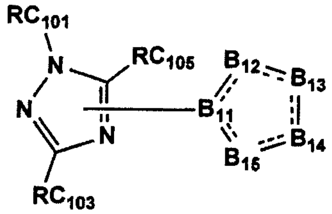

General Formula (1-D)

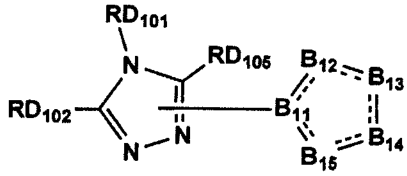

General Formula (1-E)

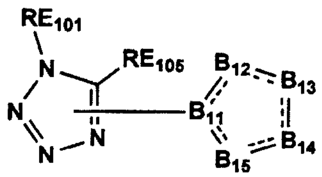

General Formula (1-F)

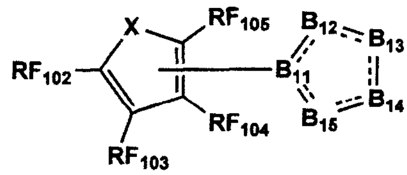

General Formula (1-G)

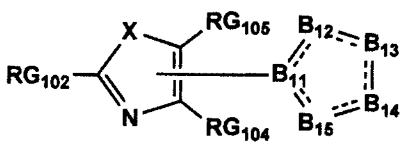

General Formula (1-H)

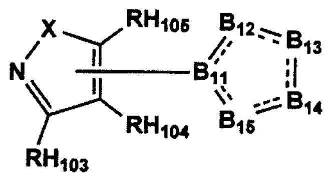

General Formula (1-I)

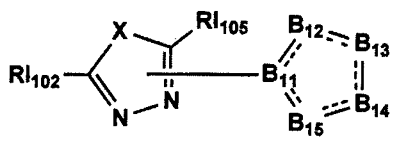

In the General Formula (1-A) to General Formula (1-I), X represents an oxygen atom or a sulfur atom; $RA_{101}$ to $RG_{105}$ each represent a linking site with $B_{11}$, a hydrogen atom or a substituent; and an arbitrary one of each of $RA_{101}$ to $RA_{105}$, $RB_{101}$ to $RB_{105}$, $RC_{101}$ to $RC_{105}$, $RD_{101}$ to $RD_{105}$, $RE_{101}$ and $RE_{105}$, $RF_{102}$ to $RF_{105}$, $RF_{102}$ to $RF_{105}$, $RH_{103}$ to $RH_{105}$, and $RI_{102}$ and $RI_{105}$ is used to be linked with $B_{11}$.

Of $RA_{101}$ to $RI_{105}$, the ones not used to be linked with $B_{11}$ each represent a hydrogen atom or a substituent, preferably a hydrogen atom, an alkyl group, an aryl group or a heteroaryl group, and far preferably a hydrogen atom, an aryl group or a heteroaryl group.

$B_{11}$ to $B_{15}$ each represent $CR_1$, a nitrogen atom, an oxygen atom or a sulfur atom, provided that at least one of $B_{11}$ to $B_{15}$ represents a nitrogen atom, an oxygen atom or a sulfur atom. A doublet of a solid line and a broken line in the ring containing $B_{11}$ represents a single bond or a double bond, and the ring containing $B_{11}$ represents an aromatic ring. $R_1$ represents a hydrogen atom or a substituent, and the substituent is preferably an alkyl group, an aryl group or a heteroaryl group, and far preferably an aryl group or a heteroaryl group. When a plurality of $R_1$ exists, the substituents may be the same or different from each other, and further may be bonded with each other to form a ring.

The structure represented by the above General Formula (1) is preferably a structure represented by the following General Formula (2).

[Chem. 10]

General Formula (2)

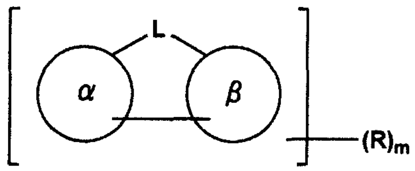

In the General Formula (2), a ring α, a ring β and R are synonymous with the ring α, ring β and R in the General Formula (1), respectively; m represents an integer of 1 to 6; and L represents a divalent linking group.

L represents a divalent linking group and links the ring α with the ring β and forms a new ring with portions of the ring α and the ring β. Examples of the linking group include an alkylene group, an alkenylene group, an ether group, an ester group, a carbonyl group, an amino group, an amide group, a silyl group, a phosphine oxide group, an arylalkylene group, a nonaromatic hydrocarbon ring, a nonaromatic heterocyclic group, -O-, -S- and a linking group formed by combination of any of these. The divalent linking group represented by L is preferably an alkylene group, an ether group, an ester group, a carbonyl group, an amino group, an amide group, a silyl group or a phosphine oxide group, far preferably an alkylene group, an ether group, an ester group, an amino group, a silyl group or a phosphine oxide group, and still far preferably an alkylene group or an ether group.

The General Formula (2) of the present invention is characterized, as described above, by having the second linkage through the linking group L in addition to the linkage of the ring α and the ring β and by forming the condensed ring of the ring α, the ring β and the ring containing the linking group L. The General Formula (2) makes it possible to have higher triplet excitation energy and higher stability as a compound than the General Formula (1) . It is assumed that owing to these features, when the material for organic EL elements represented by the General Formula (2) is used in an organic EL element, both high luminous efficiency and durability can be achieved.

The number of members of the ring newly formed through L is not particularly limited, but the ring is preferably a 5-to 10-membered ring, far preferably a 6- to 8-membered ring, and still far preferably a 7-membered ring. The reason why these numbers of members are preferred is because with these numbers of members, motility of the ring α and the ring β can be adjusted to be in the appropriate range.

The ring newly formed through L may be either an unsaturated ring or an aromatic ring, but preferably an unsaturated ring.

m represents an integer of 1 to 6. m is preferably 1 to 4, far preferably 1 to 3, and still far preferably 1 or 2.

The General Formula (2) can be a structure represented by the following General Formula (2-1).

[Chem. 11]

General Formula (2-1)

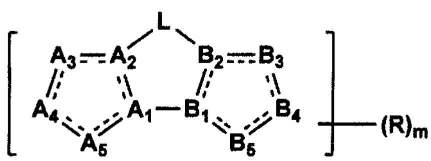

In the General Formula (2-1), $A_1$, $A_2$, $A_3$, $A_4$, $A_5$, $B_1$, $B_2$, $B_3$, $B_4$ and $B_5$ each represent a carbon atom, a nitrogen atom, an oxygen atom or a sulfur atom, and $A_1$ to $A_5$ and $B_1$ to $B_5$ respectively form 5 -membered aromatic heterocycles each derived from pyrrole, furan, thiophene, pyrazole, 1,2,3-triazole, 1,2,4-triazole, tetrazole, oxazole, isoxazole, thiazole or isothiazole; R represents a hydrogen atom or a substituent substituted at an arbitrary position of at least one of the two aromatic heterocyclic groups; m represents an integer of 1 to 8; and L represents a divalent linking group.

A doublet of a solid line and a broken line represents a single bond or a double bond, and the ring $\alpha$ formed of $A_1$ to $A_5$ and the ring $\beta$ formed of $B_1$ to $B_5$ are both aromatic rings.

R, m and L are synonymous with R, m and L in the General Formula (2), respectively. When a plurality of R exists, the substituents may be the same or different from each other, and further may be bonded with each other to form a ring. In addition, when a plurality of R exists, at least one of them is preferably a dibenzofuran group, a dibenzothiophene group, a carbazolyl group, a silyl group, a phenyl group or a phosphine oxide group, far preferably a dibenzofuran group, a dibenzothiophene group, a carbazolyl group or a silyl group, and still far preferably a dibenzofuran group, a dibenzothiophene group or a carbazolyl group.

Preferably at least one of $A_3$, $A_5$, $B_3$ and $B_5$ has a substituent, and far preferably $A_5$ or $B_5$ has a substituent. When either one of $A_3$ and $A_5$ or either one of $B_3$ and $B_5$ has a substituent, preferably, $A_4$ or $B_4$ as its vicinal part has a substituent, and the substituent is preferably an alkyl group, an aryl group or a heteroaryl group, and far preferably an alkyl group.

The General Formula (2-1) of the present invention is characterized in that linked parts by the linking group L in the General Formula (2) are put as vicinal parts of the single bond of $A_1$ and $B_1$. Making the vicinal parts of the single bond be the bonded parts with the linking group can adjust molecular motion of each of the ring formed of $A_1$ to $A_5$ and the ring formed of $B_1$ to $B_5$ to be in the appropriate range and increase stability of molecules, and also can increase triplet excitation energy. It is assumed that both high luminous efficiency and durability can be achieved thereby when the material for organic EL elements of the present invention is used in an organic EL element.

Further, the structure represented by the General Formula (2-1) can be a structure represented by the following General Formula (2-2) or General Formula (2-3).

[Chem. 12]

General Formula (2-2)

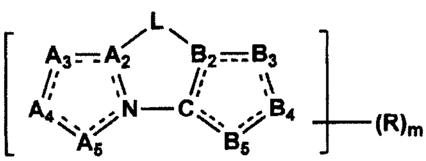

General Formula (2-3)

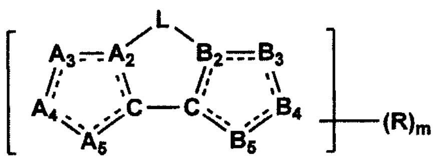

The symbols in the General Formulae (2-2) and (2-3) are synonymous with those in the above General Formula (2-1). Further, the General Formula (2) can be represented by any of the above General Formula (1-A) to General Formula (1-I).

When the General Formula (2) is represented by any of the General Formula (1-A) to General Formula (1-I), one of each of $RA_{101}$ to $RA_{105}$, $RB_{101}$ to $RB_{105}$, $RC_{101}$ to $RC_{105}$, $RD_{101}$ to $RD_{105}$, $RE_{101}$ to $RE_{105}$, $RF_{102}$ to $RF_{105}$, $RG_{102}$ to $RG_{105}$, $RH_{103}$ to $RH_{105}$, and $RI_{102}$ to $RI_{105}$ is further bonded with one of $B_{11}$ to $B_{15}$ to form a ring.

The other symbols are synonymous with those in the case where the General Formula (1) is represented by any of the General Formula (1-A) to General Formula (1-I).

Preferable examples of the substituent in the present invention include the following, and a solid line indicates a linking position. As described above, the substituent in the present invention may further have a substituent, and hence not limited to the following. In the four types on the top row, a solid line at the left end of a benzene ring indicates a linking position. In the case where two solid lines exist in a dibenzofuran group, a dibenzothiophene group or a carbazole group, the solid line at the lower left indicates a bonding position.

[Chem. 13]

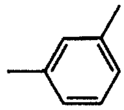 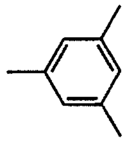 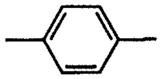 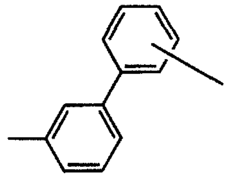

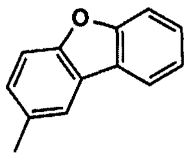 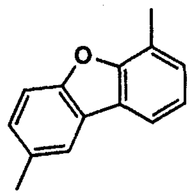 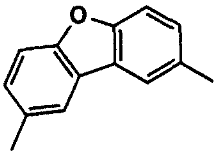 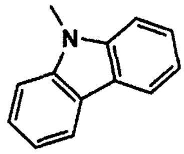

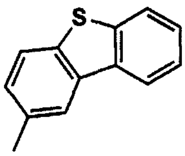 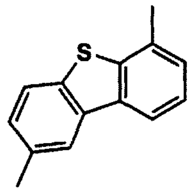 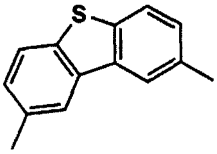 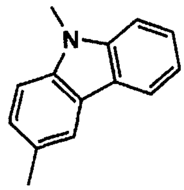

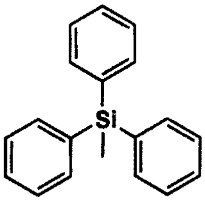 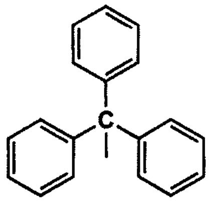 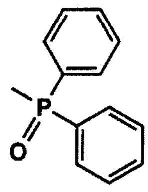 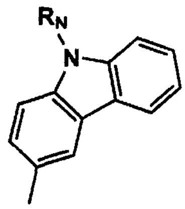

In the above formulae, $R_N$ represents a substituent which is preferably an alkyl group, an aryl group or a heteroaryl group, far preferably an aryl group or a heteroaryl group, and particularly preferably a phenyl group, a pyridyl group or a triazinyl group.

The material for organic EL elements of the present invention may be contained in any organic layer in an organic EL element, but preferably be used as a host material, a hole transport material or an electron transport material, far preferably be used as a host material or a hole transport material, and still far preferably be used as a host material together with a phosphorescent compound in the light emitting layer. Each organic layer may be composed of the compound of the present invention alone or in combination with another material. The organic layer means a layer containing an organic compound.

Tg (glass transition temperature) of the material for organic EL elements of the present invention is preferably sufficiently higher than room temperature in terms of stability over time and suitability to produce the elements, preferably 100°C or more, far preferably 120°C or more, and still far preferably 130°C or more.

The molecular weight of the material for organic EL elements of the present invention is preferably 300 or more and 2,000 or less, far preferably 500 or more and 1,500 or less, and still far preferably 700 or more and 1,250 or less.

When the compound represented by the above General Formula (1) is used together with the below-described phosphorescent compound, the compound represented by the General Formula (1) has preferably triplet excitation energy ($T_1$) higher than the phosphorescent compound, far preferably $T_1$ of 2.90 eV or more, still far preferably $T_1$ of 3.00 eV or more, and particularly preferably $T_1$ of 3.10 eV or more.

The value of the triplet excitation energy is a value calculated by using Gaussian 09, which is software for calculation of molecular orbitals produced by Gaussian, Inc. After molecular structure optimization is carried out by using B3LYP/6-31G* as a keyword, the triplet excitation energy is determined by a calculated value. The background of use of this method is because the calculated value obtained by this method and the experimental value have high correlation therebetween.

Specific examples of the compound represented by the General Formula (1) of the present invention are shown below, but the present invention is not limited thereto.

[Chem. 14]

1-1

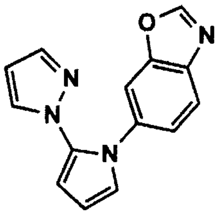

1-2

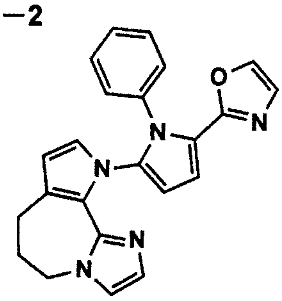

1-3

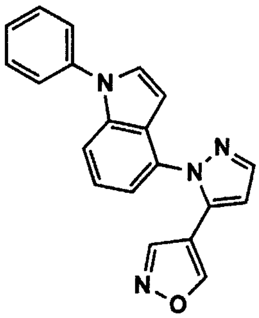

1-4

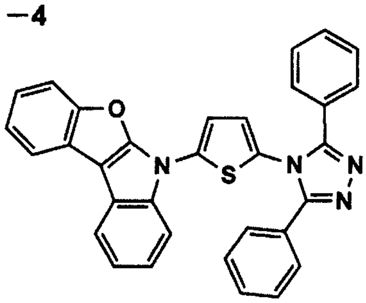

1-5

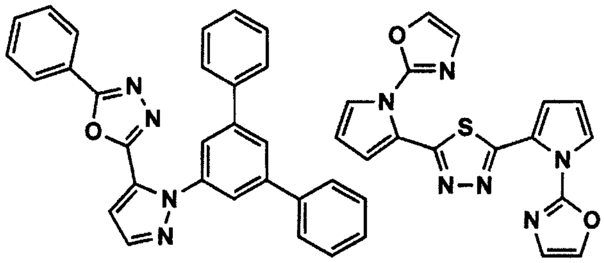

1-6

1-7

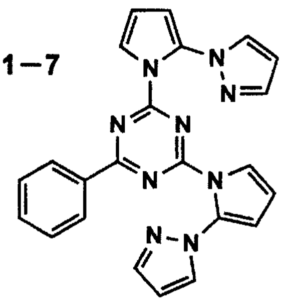

1-8

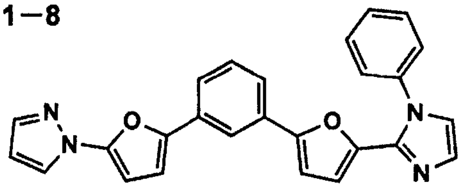

1-9

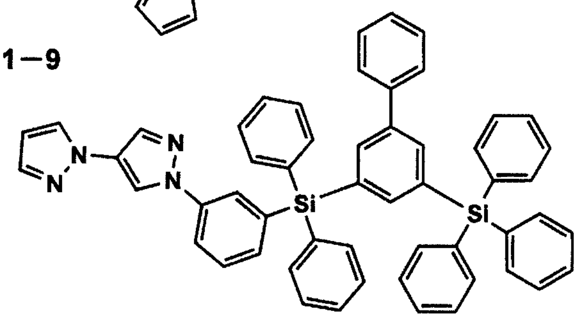

1-10

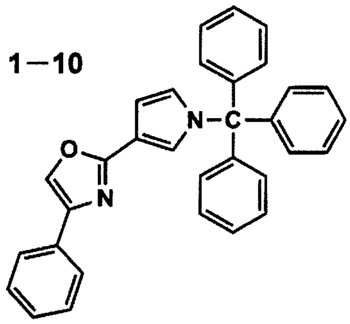

1-11

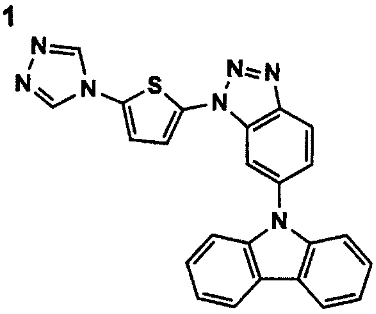

1-12

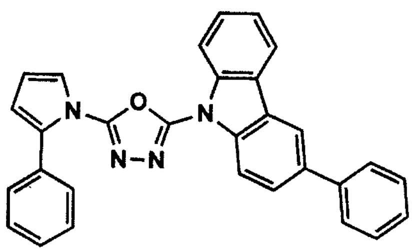

[Chem. 15]

1-13

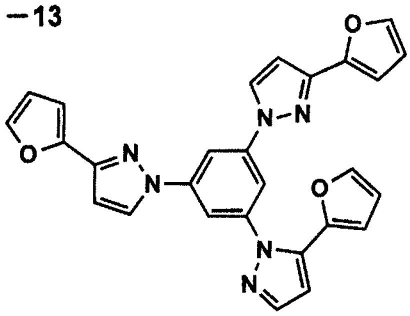

1-14

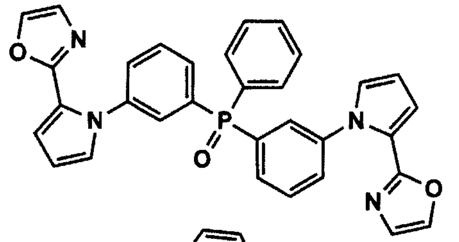

1-15

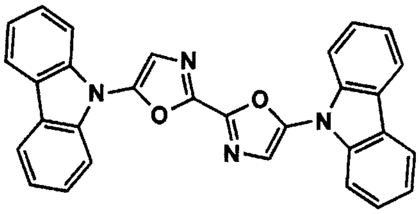

1-16

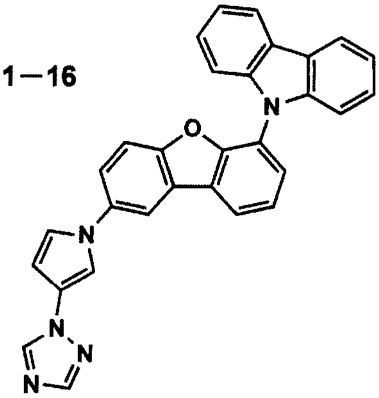

1-17

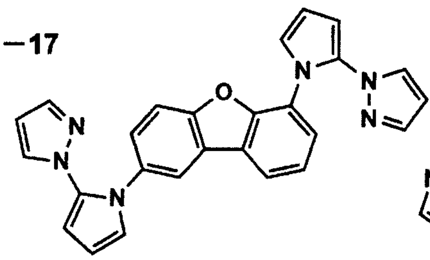

1-18

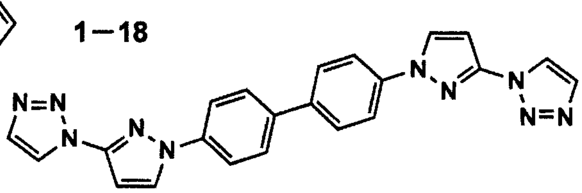

1-19

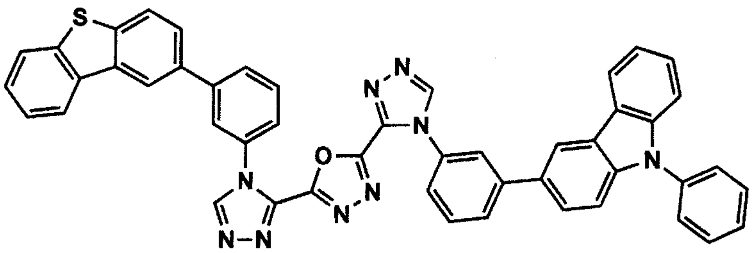

1-20

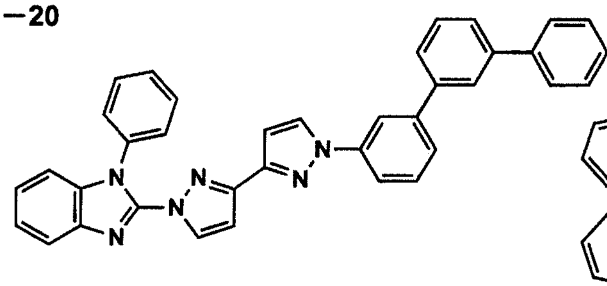

1-21

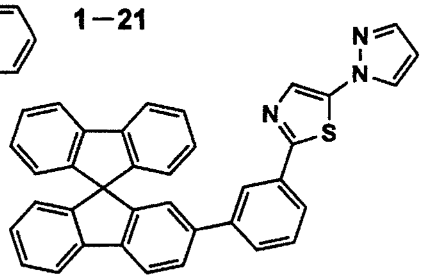

[Chem. 16]

1−22

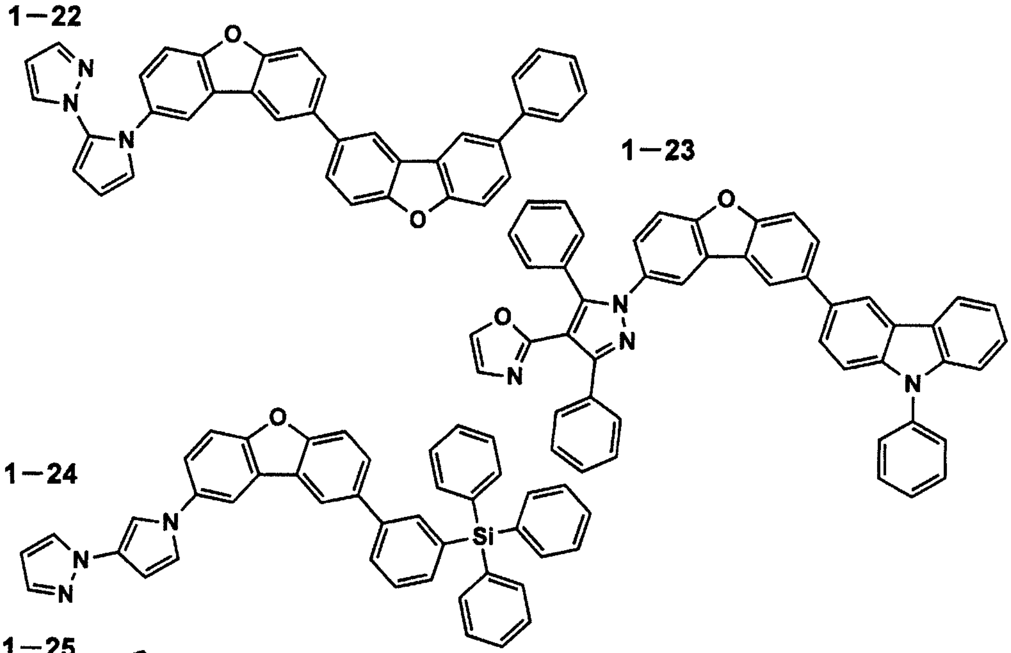

1−23

1−24

1−25

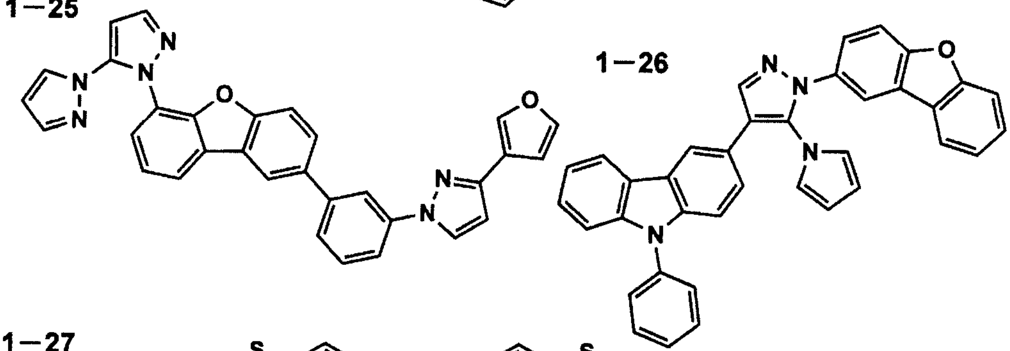

1−26

1−27

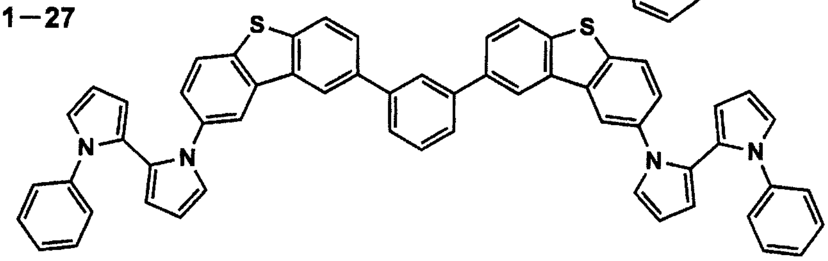

1−28

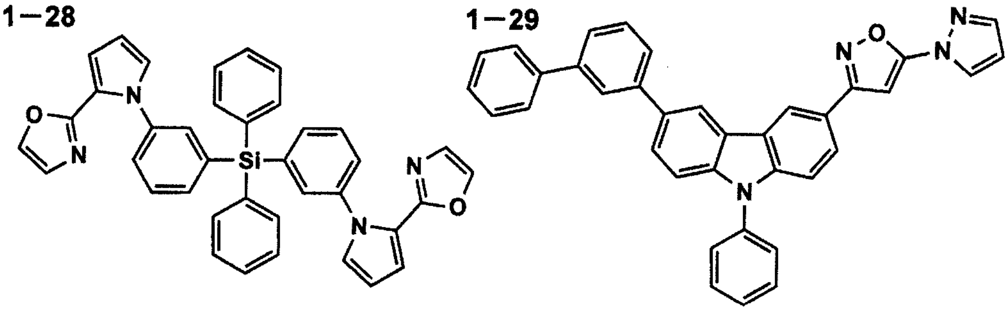

1−29

[Chem. 17]

2-1

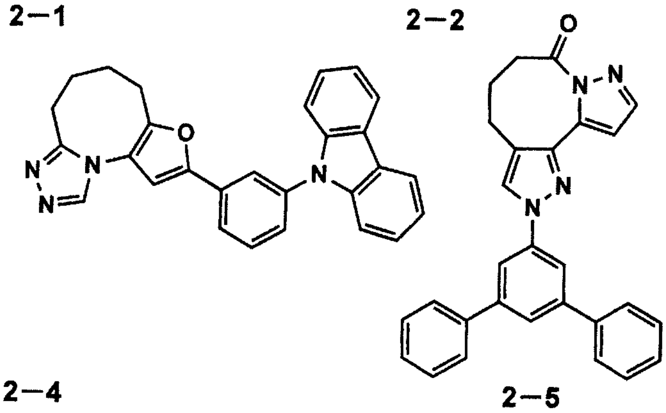

2-2

2-3

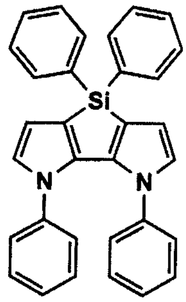

2-4

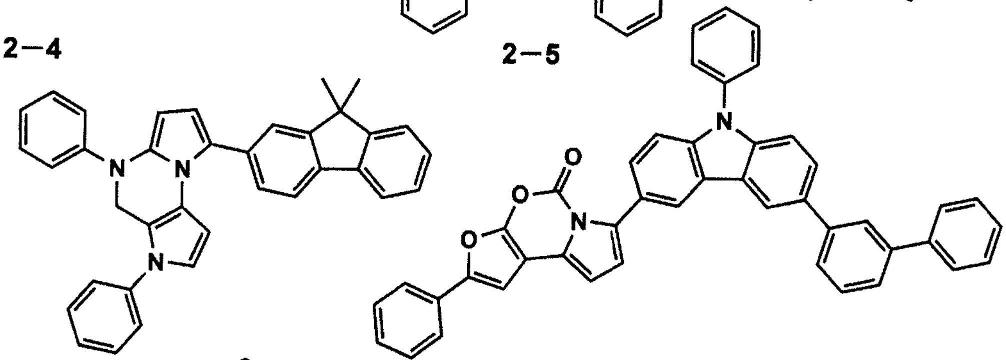

2-5

2-6

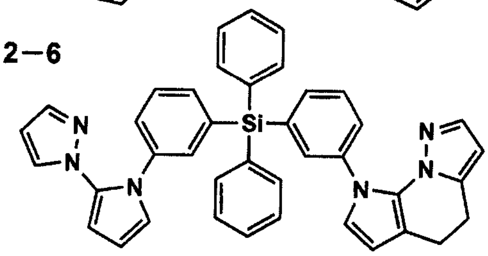

2-7

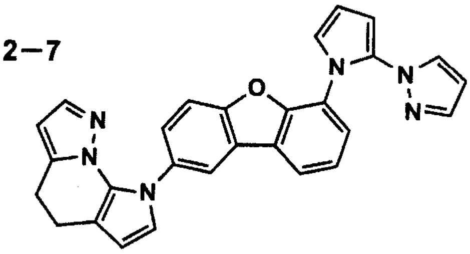

2-8

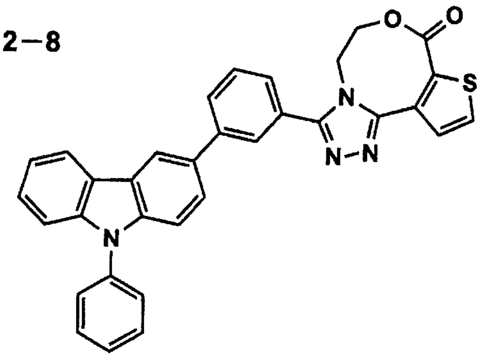

2-9

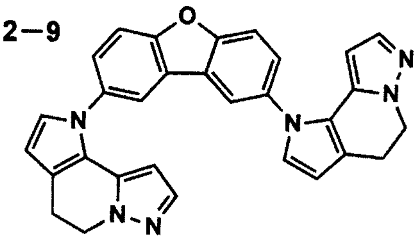

2-10

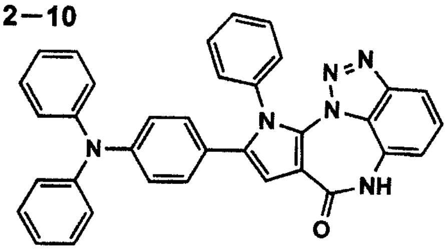

2-11

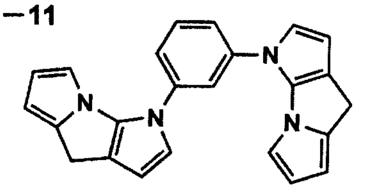

[Chem. 18]

2-12

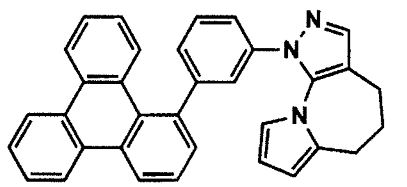

2-13

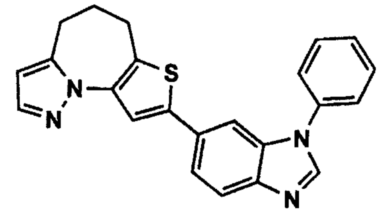

2-14

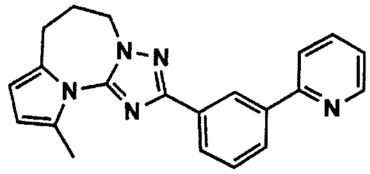

2-15

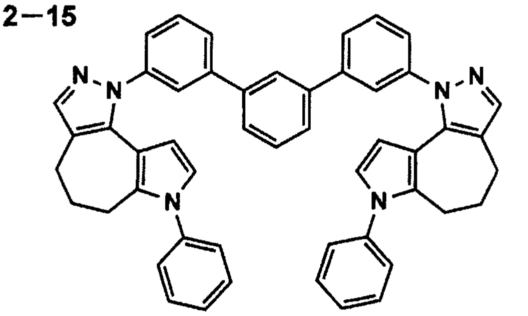

2-16

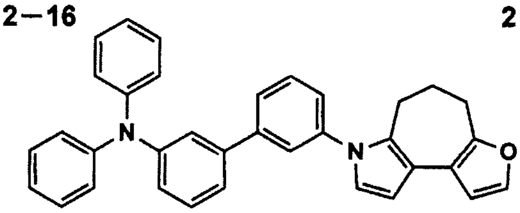

2-17

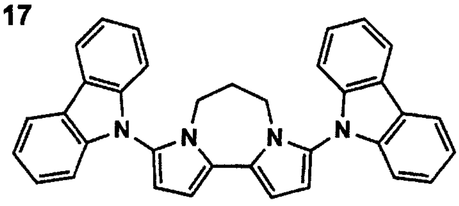

2-18

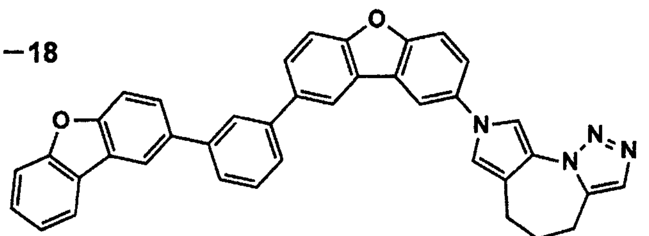

2-19

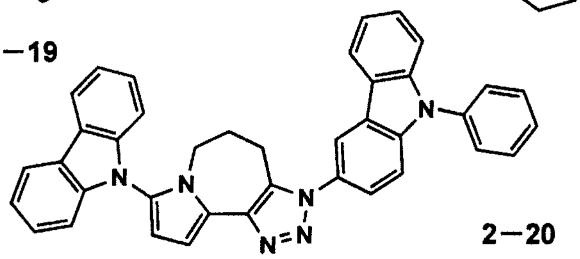

2-20

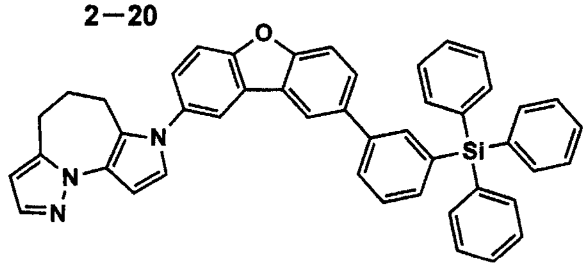

[Chem. 19]

2—21

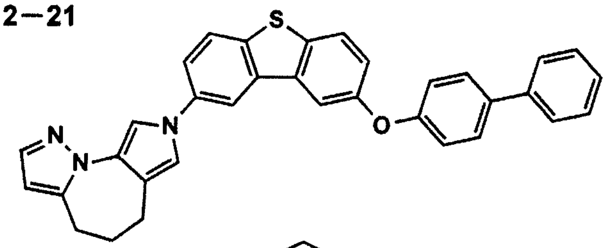

2—22

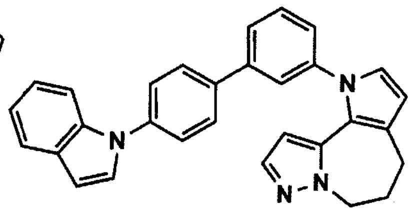

2—23

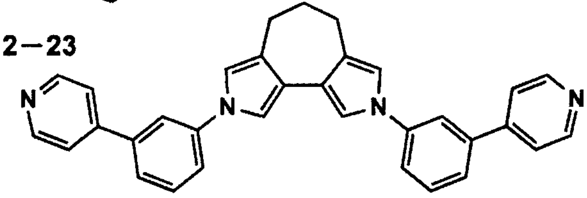

2—24

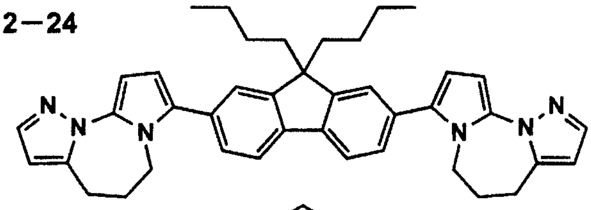

2—25

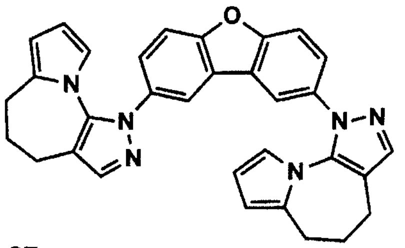

2—26

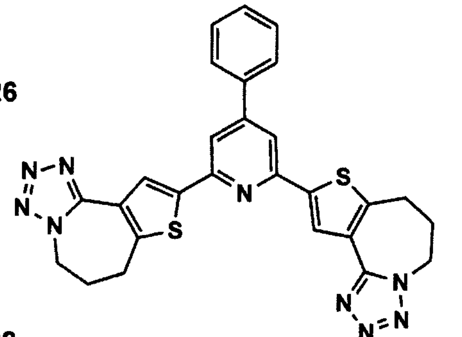

2—27

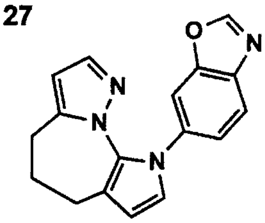

2—28

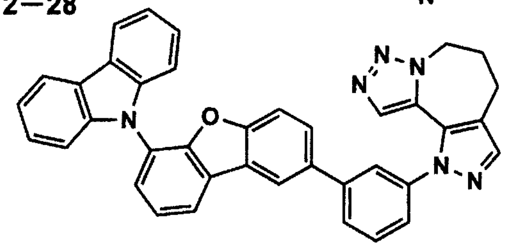

2—29

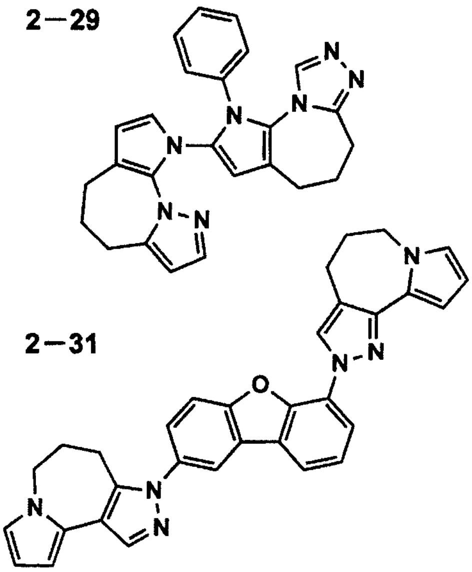

2—30

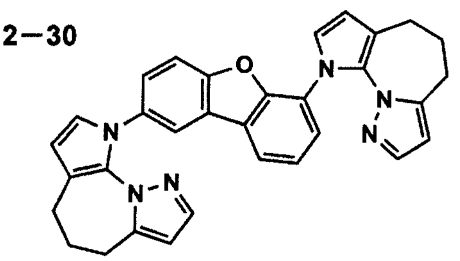

2—31

[Chem. 20]

2-32

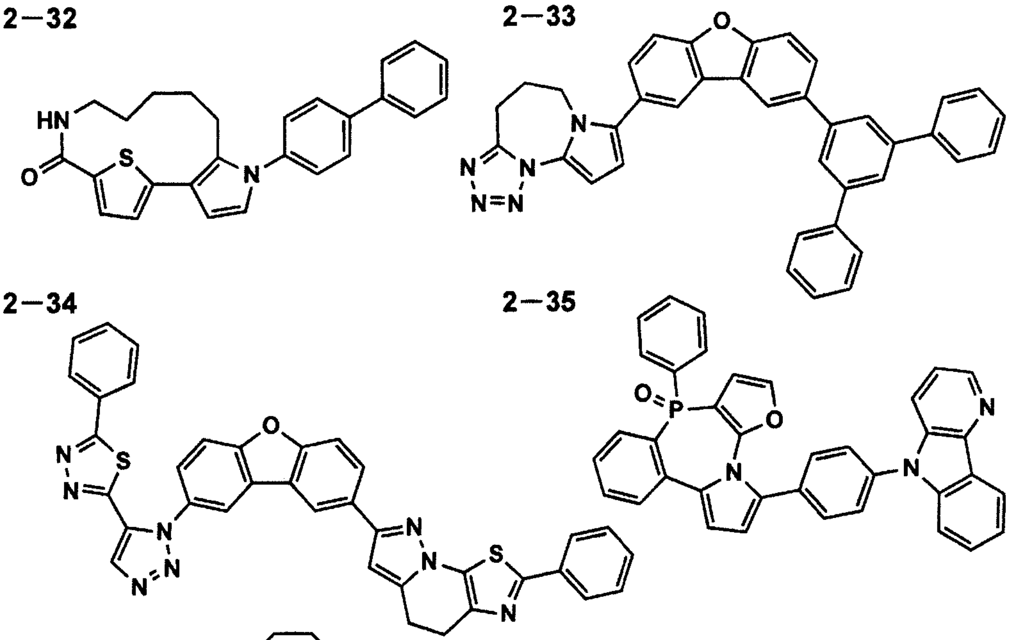

2-33

2-34

2-35

2-36

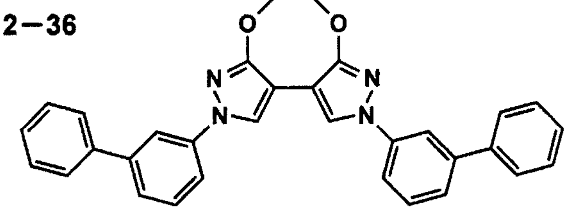

2-37

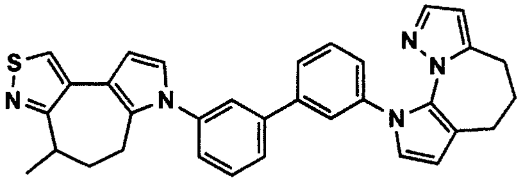

2-38

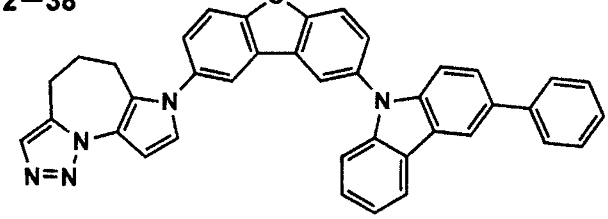

2-39

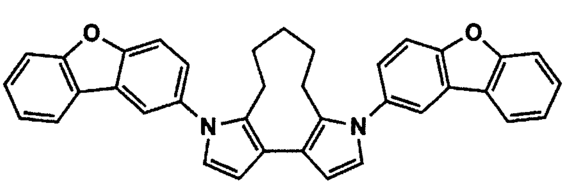

2-40

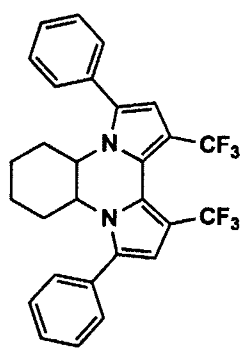

[Chem. 21]

3−1

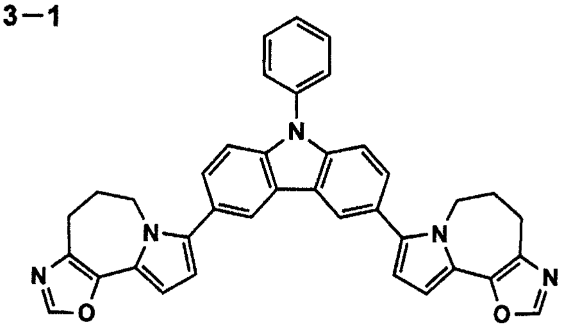

3−2

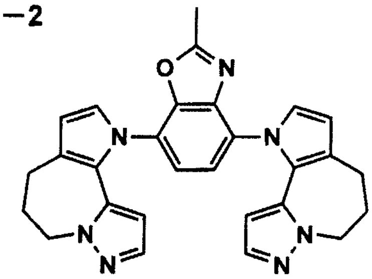

3−3

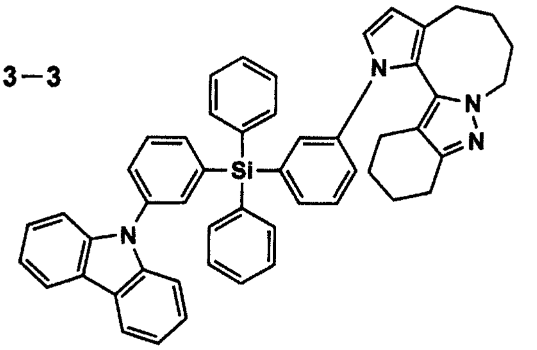

3−4

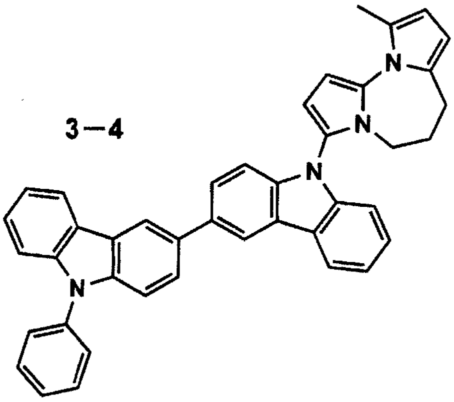

3−5

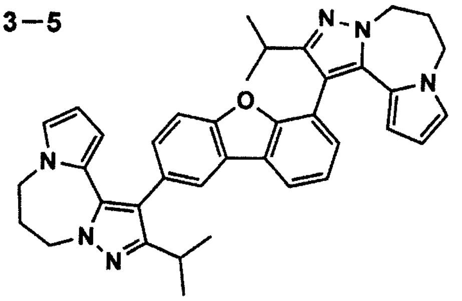

3−6

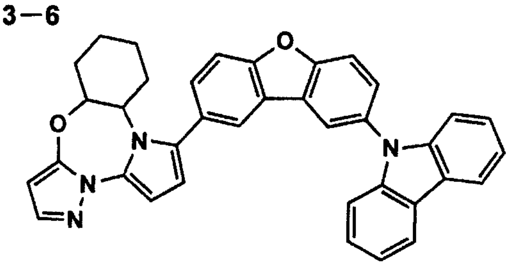

3−7

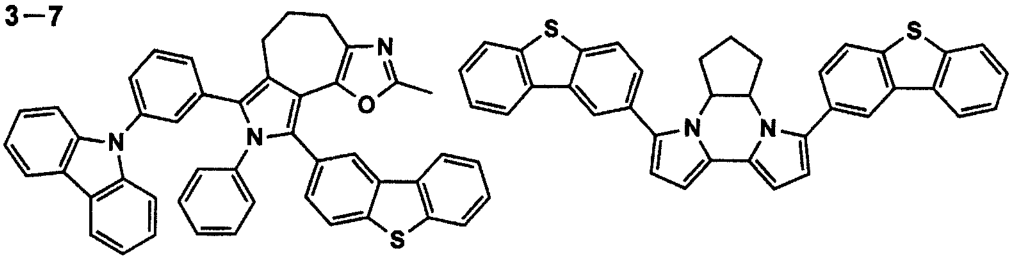

3−8

3−9

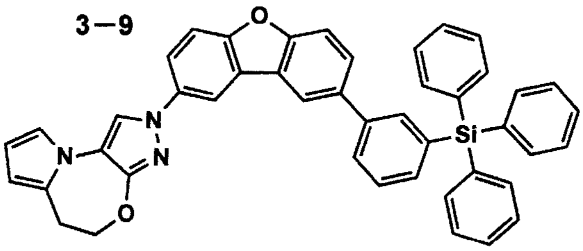

[Chem. 22]

3−10

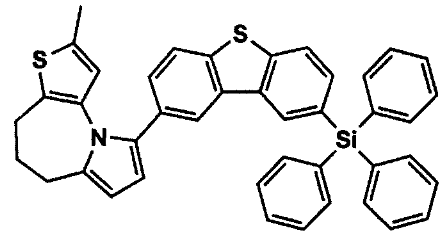

3−11

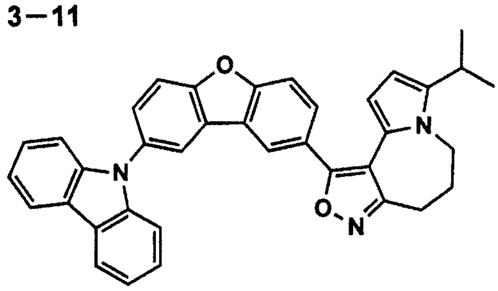

3−12

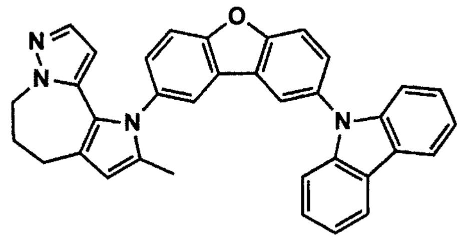

3−13

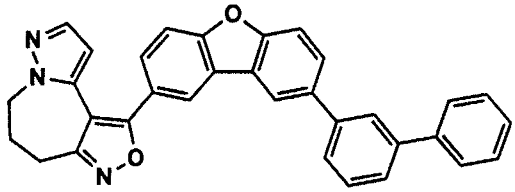

3−14

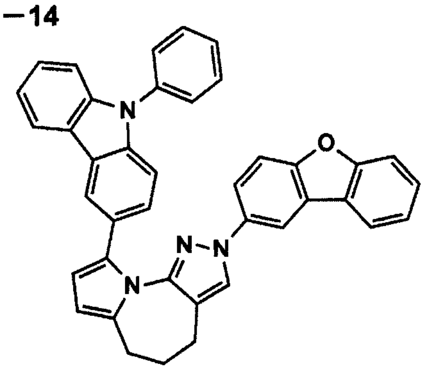

3−15

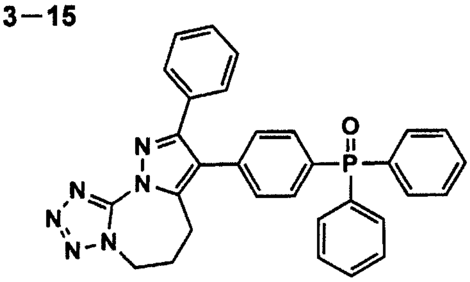

3−16

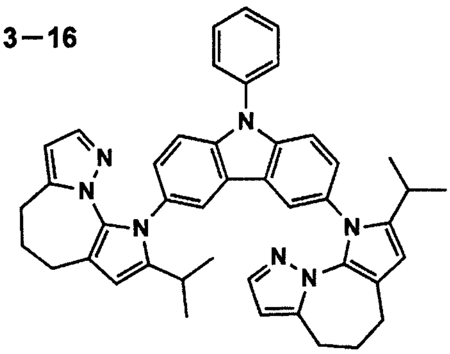

3−17

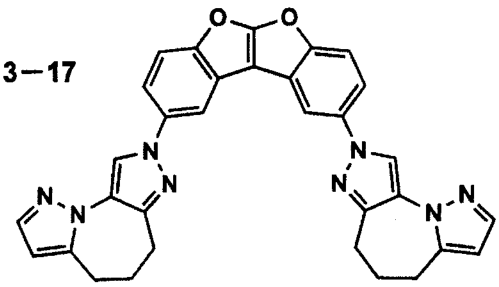

3−18

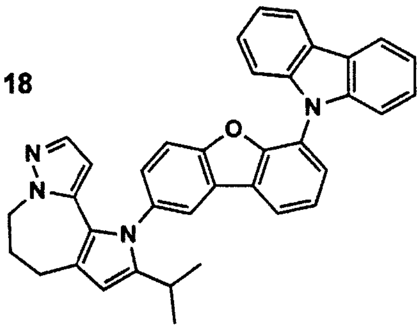

[Chem. 23]

3－19

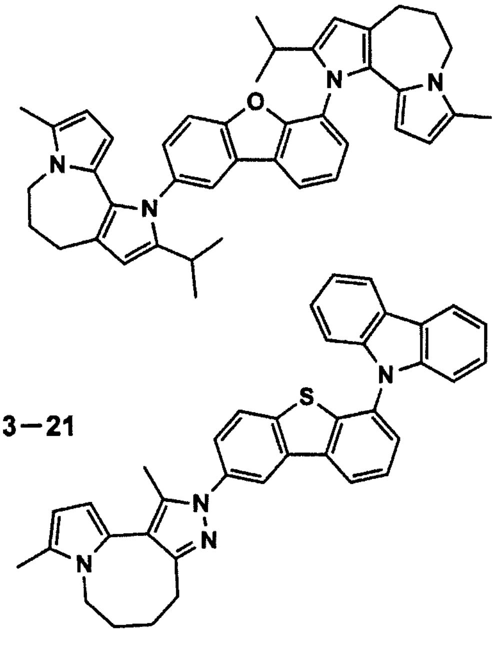

3－20

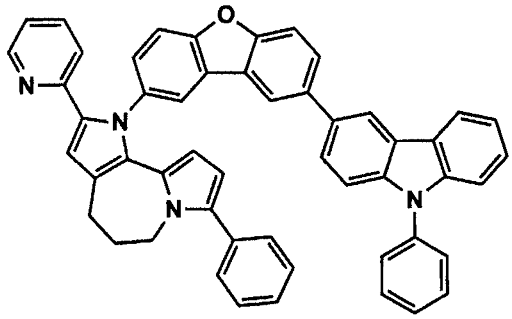

3－21

3－22

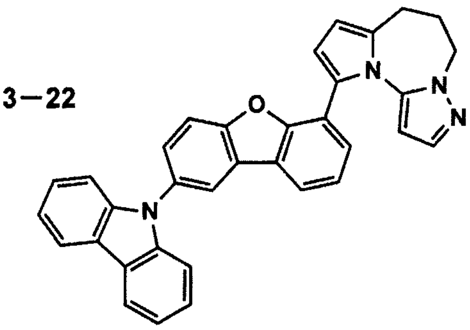

3－23

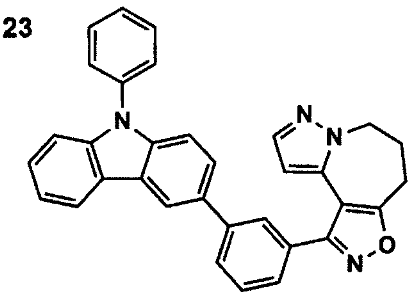

3－24

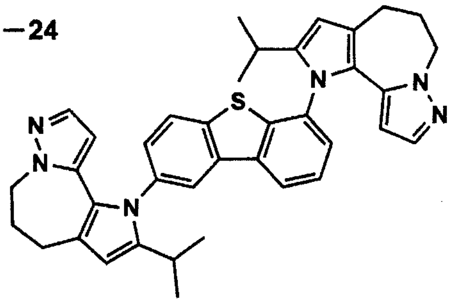

3－25

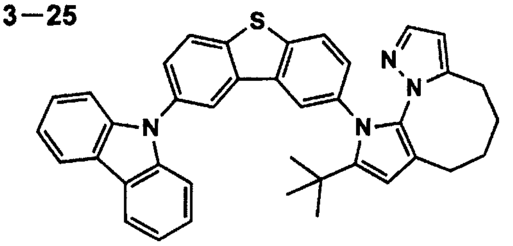

3－26

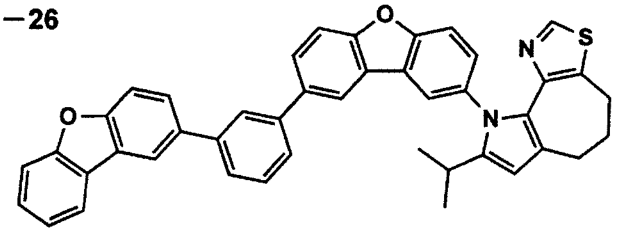

[Chem. 24]

3−27

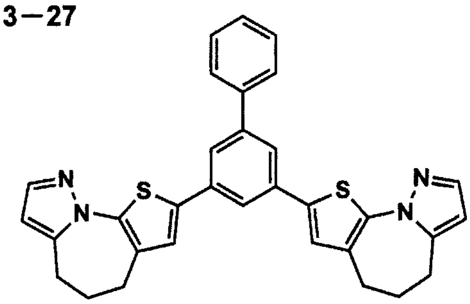

3−28

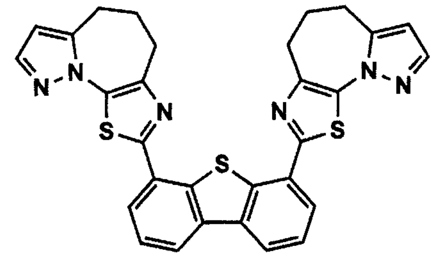

3−29

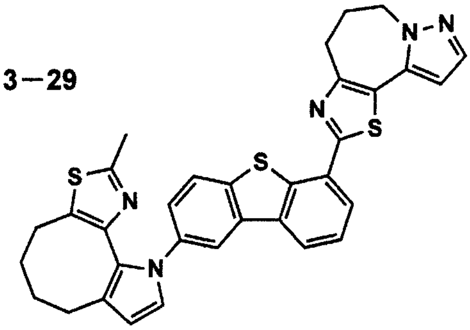

3−30

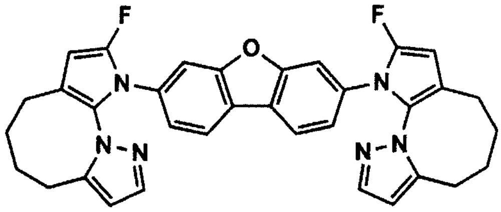

3−31

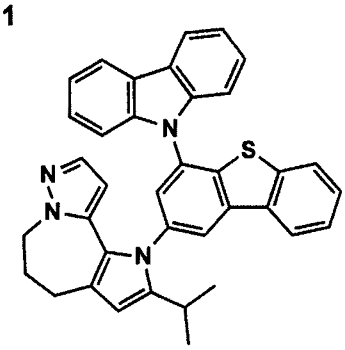

3−32

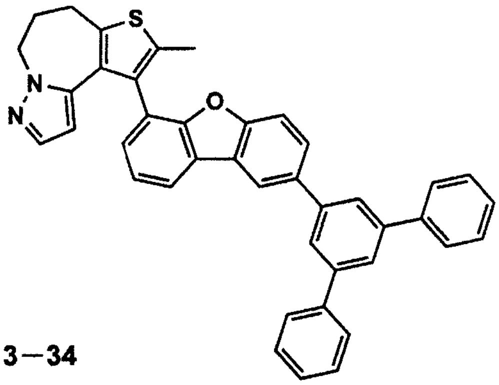

3−33

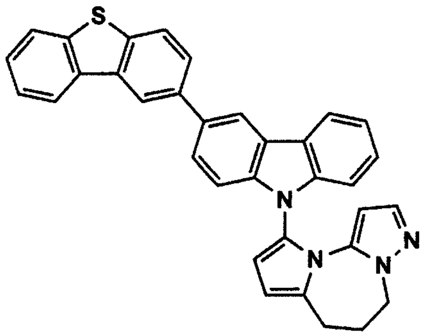

3−34

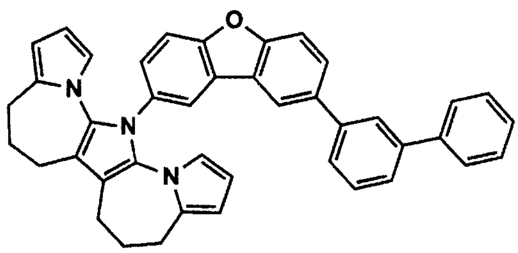

The compound represented by the General Formula (1) of the present invention can be synthesized with reference to Japanese Patent Application Publication No. 2007-23101, International Patent Application Publication No. 2012/051667, Japanese Patent No. 5076891, International Patent Application Publication No. 2011/134013 and so forth.

Hereinafter, the phosphorescent compound (phosphorescent dopant) represented by the General Formula (DP) and preferably used in the present invention is described.

[Chem. 25]

General Formula (DP)

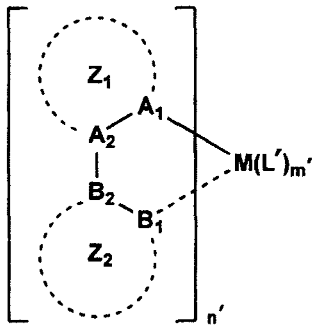

In the General Formula, M represents Ir, Pt, Rh, Ru, Ag, Cu or Os; $A_1$, $A_2$, $B_1$ and $B_2$ each represent a carbon atom or a nitrogen atom; a ring $Z_1$ represents a 6-membered aromatic hydrocarbon ring or a 5- or 6-membered aromatic heterocycle formed with $A_1$ and $A_2$; a ring $Z_2$ represents a 5- or 6-membered aromatic heterocycle formed with $B_1$ and $B_2$; the ring $Z_1$ and the ring $Z_2$ may respectively have substituents, the substituents may be bonded with each other to form a condensed ring structure, and the substituents of ligands may be bonded with each other so that the ligands are linked with each other; L' represents a monoanionic bidentate ligand coordinated to M; m' represents an integer of 0 to 2, and n' represents an integer of 1 to 3, provided that m' + n' is 2 or 3; and when n' is 2 or more, the ligands represented by the ring $Z_1$ and the ring $Z_2$ may be the same or different from each other, and when m' is 2 or more, L's may be the same or different from each other.

In the General Formula (DP), M can be Ir, Pt, Rh, Ru, Ag, Cu or Os, preferably Ir, Pt, Rh, Ru or Os, and far preferably Ir, Pt or Os.

$A_1$, $A_2$, $B_1$ and $B_2$ each represent a carbon atom or a nitrogen atom; the ring $Z_1$ represents a 6-membered aromatic hydrocarbon ring or a 5- or 6-membered aromatic heterocycle formed with $A_1$ and $A_2$; and the ring $Z_2$ represents a 5- or 6-membered aromatic heterocycle formed with $B_1$ and $B_2$.

The ring $Z_2$ is preferably a 5-membered aromatic heterocycle, and at least one of $B_1$ and $B_2$ is preferably a nitrogen atom.

The ring $Z_1$ and the ring $Z_2$ may respectively have substituents, and examples of the substituent(s) are the same as those of the substituent cited for the above General Formula (1) . The substituents of the ring $Z_1$ and/or the ring $Z_2$ may be bonded with each other to form a condensed ring structure(s). The substituents of the ligands may be bonded with each other so that the ligands are linked with each other.

L' represents a monoanionic bidentate ligand coordinated to M.

m' represents an integer of 0 to 2, and n' represents an integer of 1 to 3, provided that m' + n' is 2 or 3.

When n' is 2 or more, the ligands represented by the ring $Z_1$ and the ring $Z_2$ may be the same or different from each other, and when m' is 2 or more, L's may be the same or different from each other.

The structure represented by the General Formula (DP) is preferably a structure represented by the following General Formula (DP-1) or General Formula (DP-2).

[Chem. 26]

General Formula (DP-1)

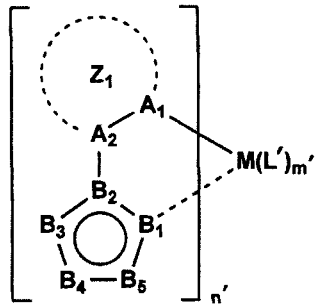

M, $A_1$, $A_2$, $B_1$, $B_2$, a ring $Z_1$, L', m' and n' in the General Formula (DP-1) are synonymous with M, $A_1$, $A_2$, $B_1$, $B_2$, the ring $Z_1$, L', m' and n' in the General Formula (DP), respectively.

$B_3$, $B_4$ and $B_5$ are a group of atoms to form an aromatic heterocycle and each represent a carbon atom, a nitrogen atom, an oxygen atom or a sulfur atom which may have a hydrogen atom or a substituent. Examples of the substituent which $B_3$, $B_4$ and $B_5$ each may have are the same as those of the substituent which the ring $Z_1$ and the ring $Z_2$ in the above General Formula (DP) each may have.

The aromatic heterocycle formed of $B_1$ to $B_5$ in the General Formula (DP-1) is preferably represented by a structure represented by any of the following General Formulae (DP-1a), (DP-1b) and (DP-1c), and far preferably represented by the structure represented by the General Formula (DP-1c).

[Chem. 27]

General Formula (DP-1a)

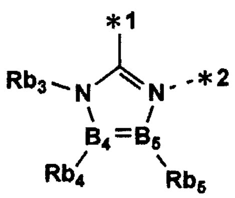

General Formula (DP-1b)

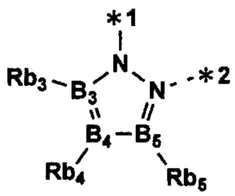

General Formula (DP-1c)

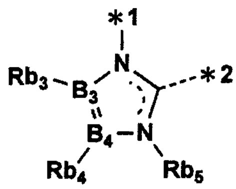

In the General Formulae (DP-1a), (DP-1b) and (DP-1c), *1 represents a bonding site with $A_2$ in the General Formula (DP-1), and *2 represents a bonding site with M therein.

$Rb_3$, $Rb_4$ and $Rb_5$ each represent a hydrogen atom or a substituent, and examples of the substituent represented by each of $Rb_3$, $Rb_4$ and $Rb_5$ are the same as those of the substituent which the ring $Z_1$ and the ring $Z_2$ in the above General Formula (DP) each may have.

In the General Formula (DP-1a), $B_4$ and $B_5$ each represent a carbon atom or a nitrogen atom, and preferably at least one of them is a carbon atom.

In the General Formula (DP-1c), $B_3$ and $B_4$ each represent a carbon atom or a nitrogen atom, preferably at least one of them is a carbon atom; far preferably the substituents represented by $Rb_3$ and $Rb_4$ are bonded with each other to form a condensed ring structure, and this newly formed condensed ring structure is preferably an aromatic ring which is preferably a benzimidazole ring, an imidazopyridine ring, an imidazopyrazine ring or a purine ring; and $Rb_5$ is preferably an alkyl group or an aryl group, and far preferably a phenyl group.

[Chem. 28]

General Formula (DP-2)

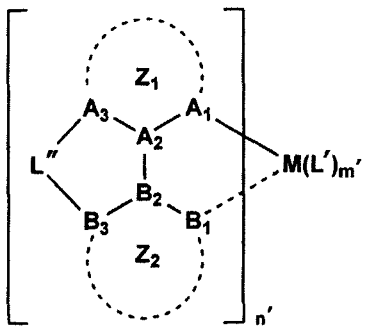

M, $A_1$, $A_2$, $B_1$, $B_2$, a ring $Z_1$, L', m' and n' in the General Formula (DP-2) are synonymous with M, $A_1$, $A_2$, $B_1$, $B_2$, the ring $Z_1$, L', m' and n' in the General Formula (DP), respectively.
The ring $Z_2$ represents a 5-membered aromatic heterocycle formed with $B_1$ to $B_3$.
$A_3$ and $B_3$ each represent a carbon atom or a nitrogen atom, and L" represents a divalent linking group. Examples of the divalent linking group represented by L" include an alkylene group, an alkenylene group, an arylene group, a heteroarylene group, a divalent heterocyclic group, -O-, -S- and a linking group formed by combination of any of these.
The General Formula (DP-2) is preferably represented by the General Formula (DP-2a).

[Chem. 29]

General Formula (DP-2a)

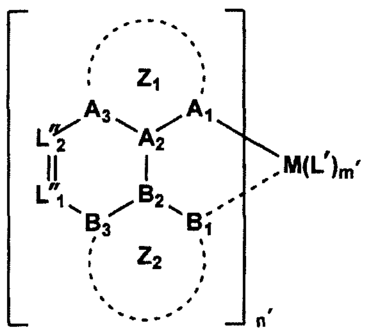

M, $A_1$, $A_2$, $B_1$, $B_2$, a ring $Z_1$, a ring $Z_2$, L', m' and n' in the General Formula (DP-2a) are synonymous with M, $A_1$, $A_2$, $B_1$, $B_2$, the ring $Z_1$, the ring $Z_2$, L', m' and n' in the General Formula (DP-2), respectively.
$L"_1$ and $L"_2$ each represent C-$Rb_6$ or a nitrogen atom, and $Rb_6$ represents a hydrogen atom or a substituent. When $L"_1$ and $L"_2$ are both C-$Rb_6$, these $Rb_6$ may be bonded with each other to form a ring.
In the General Formulae (DP), (DP-1), (DP-2) and (DP-2a), $A_2$ is preferably a carbon atom, and further $A_1$ is preferably a carbon atom. Far preferably, the ring $Z_1$ is a substituted or unsubstituted benzene ring or pyridine ring, and still far preferably, the ring $Z_1$ is a benzene ring.

<<Constituent Layers of Organic EL Element>>

Representative element structures of an organic EL element of the present invention are as follows. However, the present invention is not limited thereto.

(1) Anode/ Light Emitting Layer/ Cathode
(2) Anode/ Light Emitting Layer/ Electron Transport Layer/ Cathode
(3) Anode/ Hole Transport Layer/ Light Emitting Layer/ Cathode
(4) Anode/ Hole Transport Layer/ Light Emitting Layer/ Electron Transport Layer/ Cathode
(5) Anode/ Hole Transport Layer/ Light Emitting Layer/ Electron Transport Layer/ Electron Injection Layer/ Cathode
(6) Anode/ Hole Injection Layer/ Hole Transport Layer/ Light Emitting Layer/ Electron Transport Layer/ Cathode
(7) Anode/ Hole Injection Layer/ Hole Transport Layer/ (Electron Blocking Layer/) Light Emitting Layer/ (Hole Blocking Layer/) Electron Transport Layer/ Electron Injection Layer/ Cathode

Among the above, the structure (7) is preferably used. However, this is not a limitation.

A light emitting layer of the present invention is composed of a single layer or a plurality of layers. When the light emitting layer is composed of a plurality of layers, a non-luminescent intermediate layer (s) may be disposed between light emitting layers.

According to necessity, a hole blocking layer (also called a hole barrier layer) and/or an electron injection layer (also called a cathode buffer layer) may be disposed between the light emitting layer and a cathode. Further, an electron blocking layer (also called an electron barrier layer) and/or a hole injection layer (also called an anode buffer layer) may be disposed between the light emitting layer and an anode.

An electron transport layer of the present invention is a layer having a function of transporting electrons. The electron injection layer and the hole blocking layer are kinds of the electron transport layer in a broad sense. The electron transport layer may be composed of a plurality of layers.

A hole transport layer of the present invention is a layer having a function of transporting holes. The hole injection layer and the electron blocking layer are kinds of the hole transport layer in a broad sense. The hole transport layer may be composed of a plurality of layers.

In each of the above representative element structures, the layers except the anode and the cathode are called "organic layers".

(Tandem Structure)

An organic EL element of the present invention may be so-called a tandem structure element in which light emitting units each containing at least one light emitting layer are laminated.

A representative element structure of the tandem structure element is, for example, as follows.

Anode/ First Light Emitting Unit/ Intermediate Layer/ Second Light Emitting Unit/ Intermediate Layer/ Third Light Emitting Unit/ Cathode

All the light emitting units may be the same or different from each other, or two light emitting units may be the same with the remaining one light emitting unit different therefrom. The light emitting units may be laminated directly or may be laminated through an intermediate layer(s) (an intermediate electrode, an intermediate conductive layer, a charge generating layer, an electron drawing layer, a connecting layer or an intermediate insulating layer), and any known material structure can be used as long as a layer has a function of supplying electrons to an adjacent layer on the anode side and holes to an adjacent layer on the cathode side.

Examples of a material used for the intermediate layer include: conductive inorganic compound layers of, for example, ITO (indium tin oxide), IZO (indium zinc oxide), $ZnO_2$, TiN, ZrN, HfN, TiOx, VOx, CuI, InN, GaN, $CuAlO_2$, $CuGaO_2$, $SrCu_2O_2$, $LaB_6$, $RuO_2$ and Al; two-layer films or multilayer films of any of these conductive inorganic compounds; conductive organic substance layers of, for example, fullerenes such as fullerene C60 and oligothiophene; and conductive organic compound layers of, for example, metal phthalocyanines, metal-free phthalocyanines and porphyrins. However, the present invention is not limited thereto.

Examples of a preferable structure in the light emitting unit include the above representative element structures (1) to (7) from each of which the anode and the cathode are removed. However, the present invention is not limited thereto.

As specific examples of the tandem organic EL element, the element structures, the constituent materials and the like are described, for example, inU.S. Patent Nos. 7420203, 7473923, 6872472, 6107734 and 6337492, Japanese Patent Application Publication Nos. 2011-96679, 2010-192719, 2009-076929, 2008-078414 and 2007-059848, and International Patent Application Publication No. 2005/094130. However, the present invention is not limited thereto.

Hereinafter, the layers constituting an organic EL element of the present invention are described.

«Light Emitting Layer»

The light emitting layer of the present invention is a layer which provides a place of light emission via excitons produced by recombination of electrons and holes injected from the electrodes or the adjacent layers. The luminescent portion may be either in the light emitting layer or at an interface between the light emitting layer and the adjacent layer. The structure of the light emitting layer of the present invention is not particularly limited as long as it satisfies the requirements defined by the present invention.

The total thickness of the light emitting layer (s) is not particularly limited, but is adjusted to be in preferably the range from 2 nm to 5 μm, far preferably the range from 2 nm to 500 nm, and still far preferably the range from 5 nm to 200 nm in terms of homogeneity of layers formed, prevention of application of an unnecessarily high voltage during light emission, and increase in stability of emission colors against drive current.

The thickness of each light emitting layer is adjusted to be in preferably the range from 2 nm to 1 μm, far preferably the range from 2 nm to 200 nm, and still far preferably the range from 3 nm to 150 nm.

It is preferable that the light emitting layer contain a luminescent dopant (also called a luminescent dopant

compound or a dopant compound, or simply called a dopant) and a host compound (a matrix material or a luminescent host compound, or simply called a host).

(1) Luminescent Dopant

The luminescent dopant of the present invention is described.
As the luminescent dopant, it is preferable to use a fluorescent dopant (also called a fluorescent compound or the like) and a phosphorescent dopant (also called a phosphorescent material or the like). In the present invention, it is preferable that at least one light emitting layer contain a phosphorescent dopant.
The concentration of the luminescent dopant in the light emitting layer can be appropriately decided, and may be uniform in a thickness direction of the light emitting layer or have any concentration distribution. As the luminescent dopant of the present invention, multiple types thereof may be used together, and therefore a combination of dopants having different structures or a combination of a fluorescent dopant and a phosphorescent dopant may be used. Any emission color can be obtained thereby.
Emission colors of an organic EL element of the present invention or the compound of the present invention are determined by applying results obtained with a CS-2000 Spectroradiometer (produced by Konica Minolta Sensing Inc.) to the CIE chromaticity coordinates in FIG. 4.16 on page 108 of "Shinpen Shikisai Kagaku Handobukku (New Edition Handbook of Color Science)" (edited by The Color Science Association of Japan, University of Tokyo Press, 1985).
In the present invention, it is preferable that one or more light emitting layers contain luminescent dopants having different emission colors so that white light is emitted. A combination of luminescent dopants emitting white light is not particularly limited, but examples thereof include combinations of: blue and orange; and blue, green and red.
It is preferable that the "white" in an organic EL element of the present invention show chromaticity in the region of x = 0.39 $\pm$ 0.09 and y = 0.38 $\pm$ 0.08 in the CIE 1931 Color Specification System at 1,000 cd/$m^2$, when 2-degree viewing angle front luminance is measured by the above method.

(1.1) Fluorescent Dopant

The fluorescent dopant of the present invention is described.
The fluorescent dopant of the present invention is a compound which is capable of emitting light from an excited singlet. The fluorescent dopant is not particularly limited as long as light emission from an excited singlet is observed.
Examples of the fluorescent dopant of the present invention include an anthracene derivative, a pyrene derivative, a chrysene derivative, a fluoranthene derivative, a perylene derivative, a fluorene derivative, an arylacetylene derivative, a styrylarylene derivative, a styrylamine derivative, an arylamine derivative, a boron complex, a squarylium derivative, an oxobenzanthracene derivative, a fluorescein derivative, a perylene derivative, a polythiophene derivative and a rare earth complex compound.
In recent years, luminescent dopants making use of delayed fluorescence have been developed, and these may be used. Specific examples of the luminescent dopants making use of delayed fluorescence are compounds described, for example, in International Patent Application Publication No. 2011/156793, and Japanese Patent Application Publication Nos. 2011-213643 and 2010-93181. However, the present invention is not limited thereto.

(1.2) Phosphorescent Dopant

The phosphorescent dopant of the present invention is described.
The phosphorescent dopant of the present invention is a compound which emits light from an excited triplet and, to be more specific, a compound which emits phosphorescence at room temperature (25°C) and exhibits a phosphorescence quantum yield of 0.01 or more at 25°C. The phosphorescence quantum yield thereat is preferably 0.1 or more.
The phosphorescence quantum yield can be measured by a method described on page 398 of Bunko II of Dai 4 Han Jikken Kagaku Koza 7 (Spectroscopy II of Lecture of Experimental Chemistry vol. 7, 4th edition) (1992, published by Maruzen Co. , Ltd.). The phosphorescence quantum yield in a solution can be measured by using various solvents. However, it is only necessary for the phosphorescent dopant of the present invention to exhibit the above phosphorescence quantum yield (0.01 or more) by using one of appropriate solvents.
As principles regarding light emission of the phosphorescent dopant, two types are cited. One is an energy transfer type, wherein carriers recombine on a host compound to which the carriers are transferred so as to produce an excited state of the host compound, this energy is transferred to a phosphorescent dopant, and hence light emission from the phosphorescent dopant is carried out. The other is a carrier trap type, wherein a phosphorescent dopant serves as a carrier trap, carriers recombine on the phosphorescent dopant, and hence light emission from the phosphorescent dopant is carried out. In either case, the excited state energy of the phosphorescent dopant is required to be lower than

that of the host compound.

The phosphorescent dopant can be appropriately selected and used from the known phosphorescent dopants used for light emitting layers of organic EL elements.

Specific examples of the known phosphorescent dopants usable in the present invention include compounds described in the following documents.

The documents include: Nature 395, 151 (1998); Adv. Mater. 19, 739 (2007); Adv. Mater. 17, 1059 (2005); International Patent Application Publication No. 2009/100991; U.S. Patent Application Publication Nos. 2006/835469, 2006/0202194 and 2007/0087321; Inorg. Chem. 40, 1704 (2001); Chem. Mater. 16, 2480 (2004); Adv. Mater. 16, 2003 (2004) ; Appl. Phys. Lett. 86, 153505 (2005); Inorg. Chem. 42, 1248 (2003); International Patent Application Publication Nos. 2009/050290 and 2009/000673; U.S. Patent Application Publication Nos. 2009/0108737, 2009/0039776, 2009/0165846, 2008/0015355 and 2006/0263635; U.S. Patent No. 7090928; Angew. Chem. Int. Ed. 47, 1 (2008); Chem. Mater. 18, 5119 (2006); Inorg. Chem. 46, 4308 (2007); Appl. Phys. Lett. 74, 1361 (1999); International Patent Application Publication Nos. 2006/009024, 2006/056418, 2005/019373 and 2005/123873; U.S. Patent Application Publication Nos. 2006/0251923, 2005/0260441, 2007/0190359, 2008/0297033 and 2006/103874; International Patent Application Publication Nos. 2010/032663, 2008/140115, 2011/134013, 2011/157339, 2010/086089, 2009/113646, 2012/020327, 2011/051404 and 2011/073149; Japanese Patent Application Publication No. 2012-069737; Japanese Patent Application No. 2011-181303; and Japanese Patent Application Publication Nos. 2009-114086, 2003-81988, 2002-302671 and 2002-363552.

Of these, preferable phosphorescent dopants include an organic metal complex having Ir as central metal, and far preferably a complex containing a metal-carbon bond or a metal-nitrogen bond as one coordination mode.

Specific examples of the known phosphorescent dopants usable in the present invention are shown below, but the present invention is not limited thereto.

[Chem. 30]

D−1

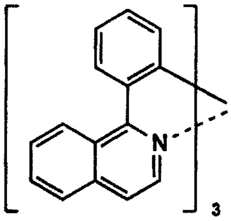

D−2

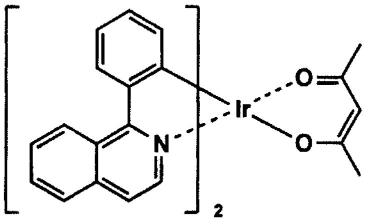

D−3

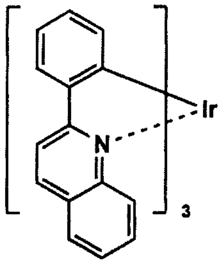

D−4

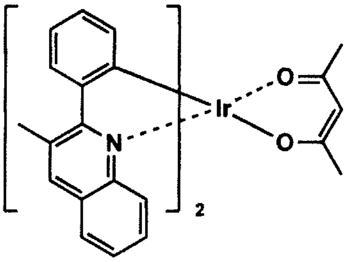

D−5

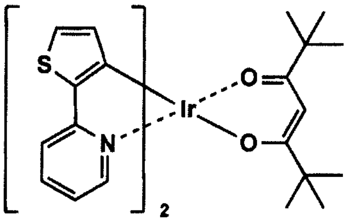

D−6

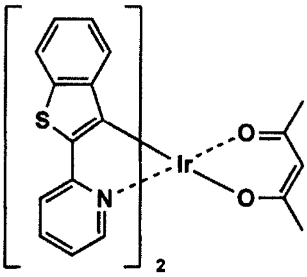

D−7

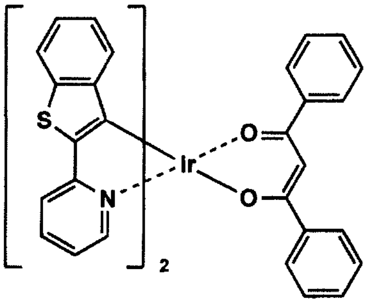

D−8

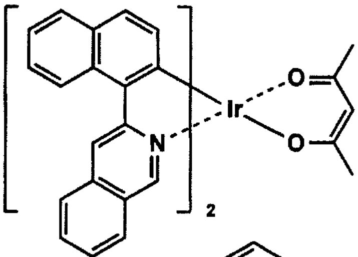

D−9

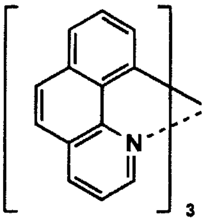

D−10

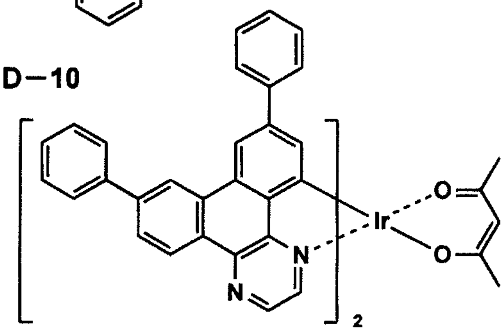

D−11

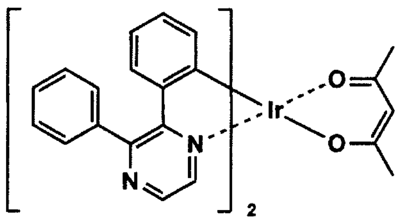

[Chem. 31]

D−12

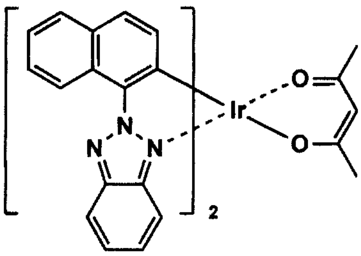

D−13

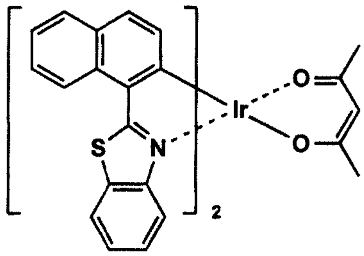

D−14

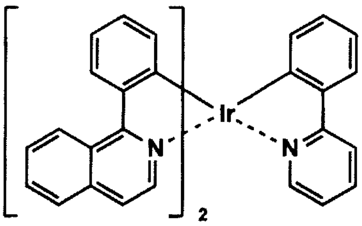

D−15

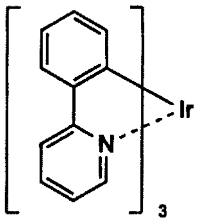

D−16

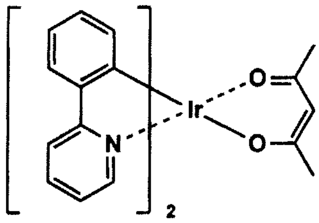

D−17

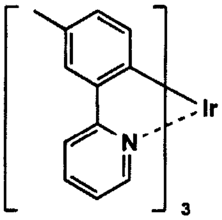

D−18

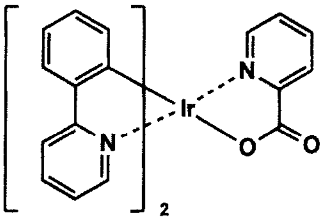

D−19

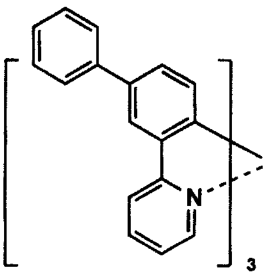

D−20

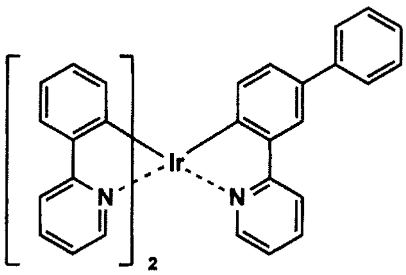

D−21

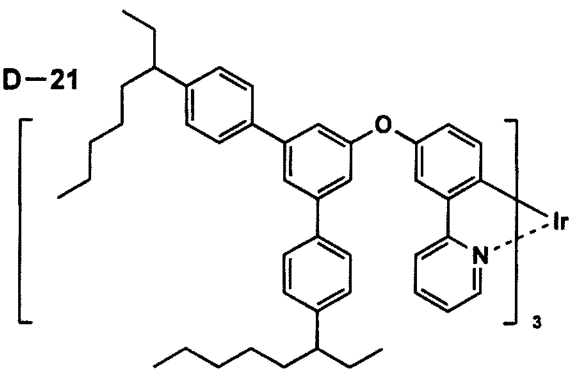

D−22

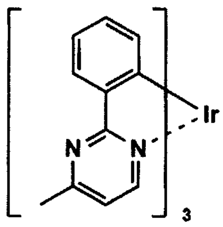

[Chem. 32]

D−23

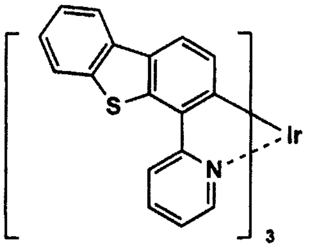

D−24

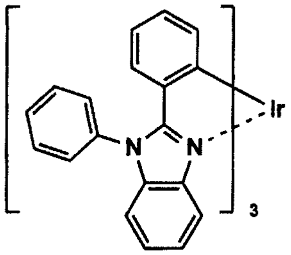

D−25

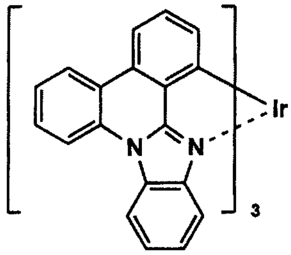

D−26

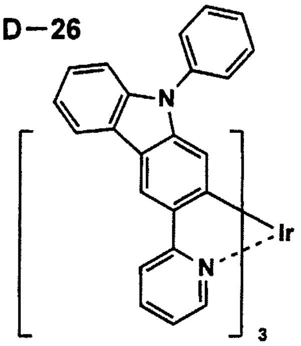

D−27

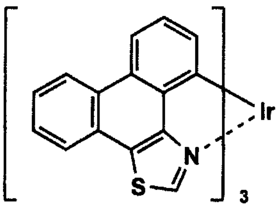

D−28

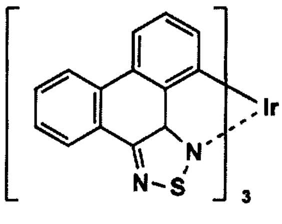

D−29

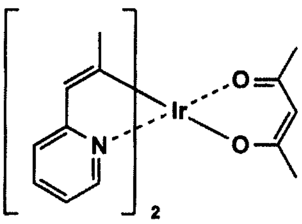

D−30

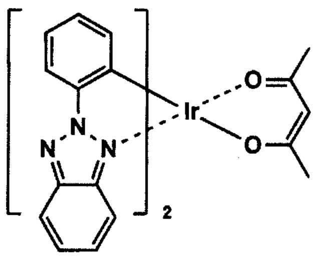

D−31

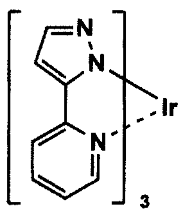

D−32

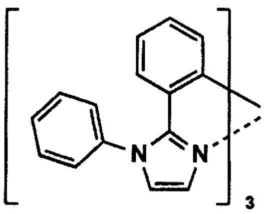

D−33

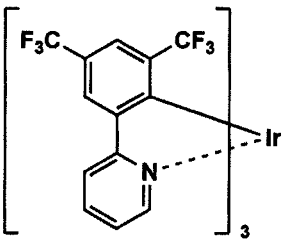

D−34

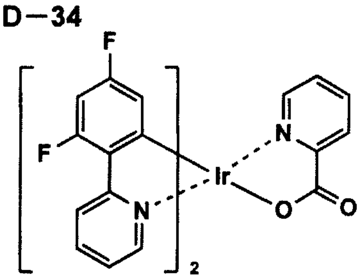

D−35

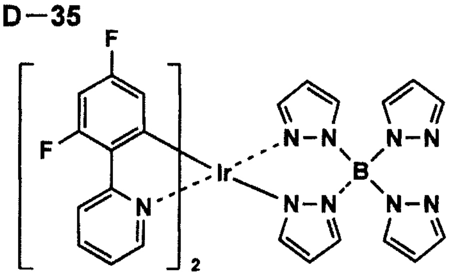

D−36

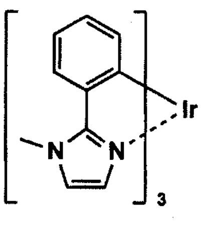

[Chem. 33]

D−37

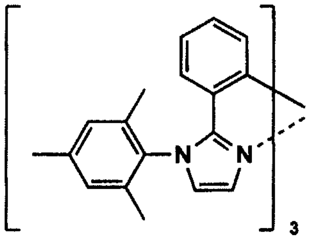

D−38

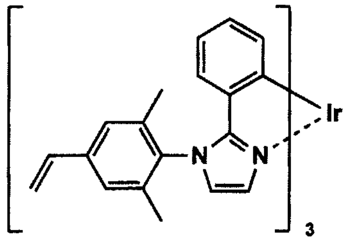

D−39

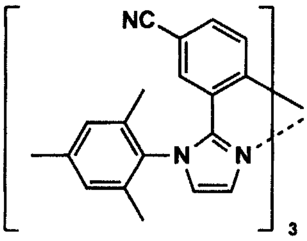

D−40

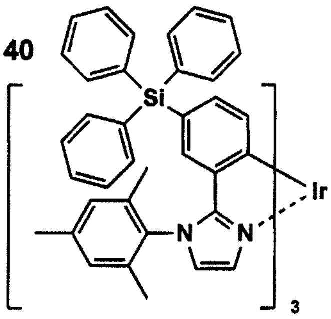

D−41

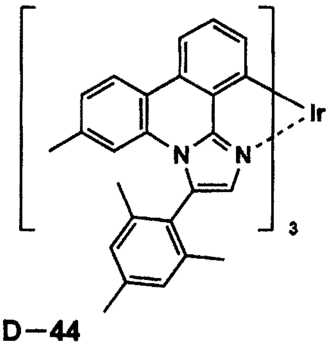

D−42

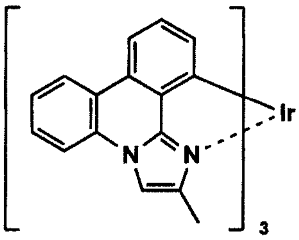

D−43

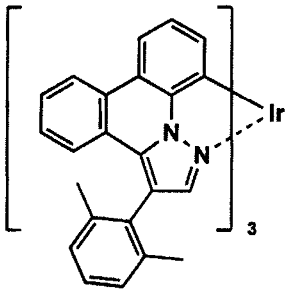

D−44

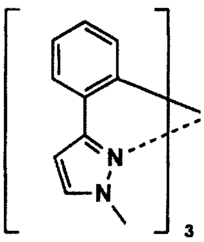

D−45

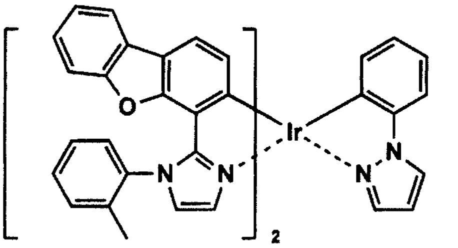

D−46

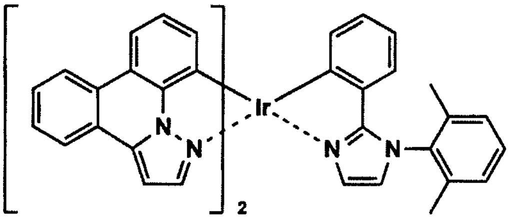

D−47

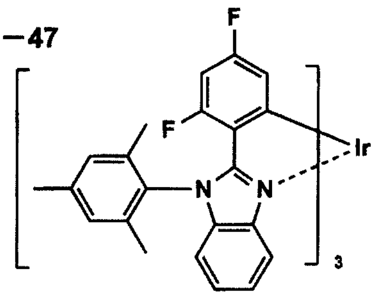

[Chem. 34]

D−48

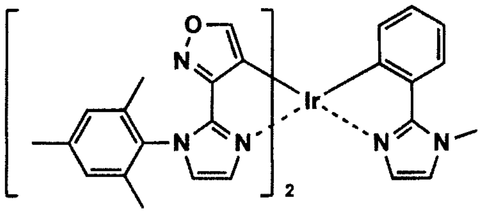

D−49

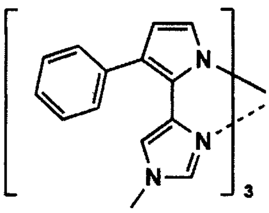

D−50

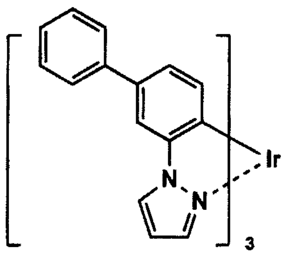

D−51

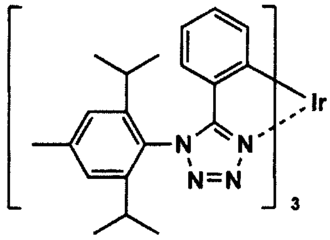

D−52

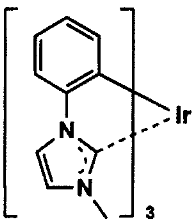

D−53

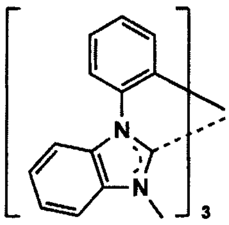

D−54

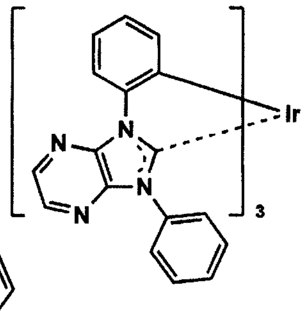

D−55

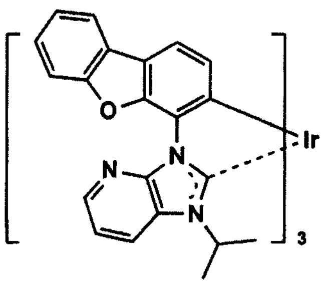

D−56

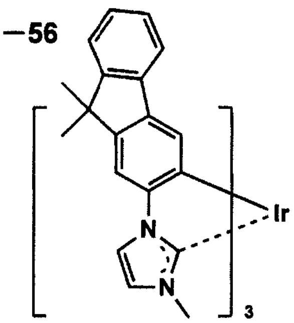

D−57

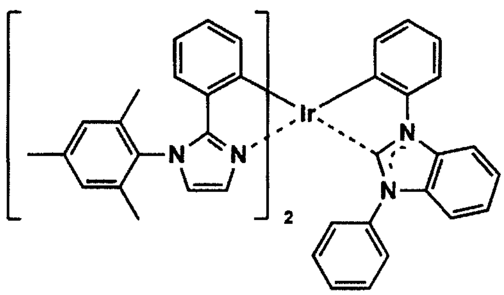

D−58

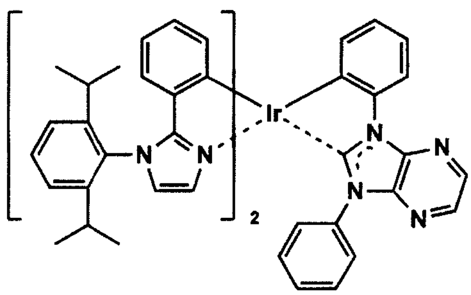

[Chem. 35]

D-59

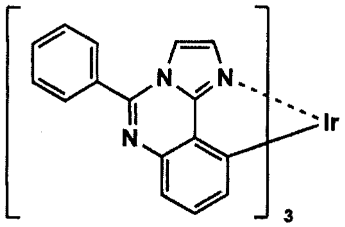

D-60

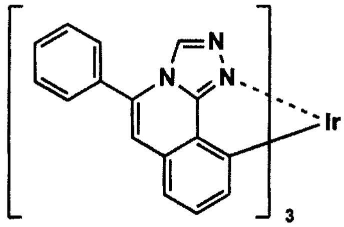

D-61

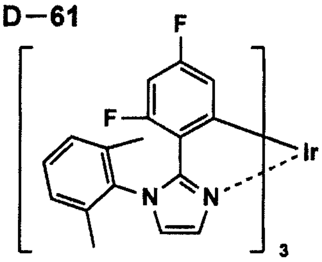

D-62

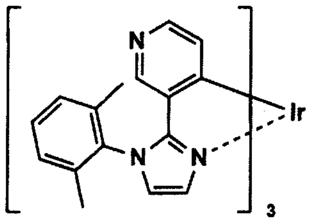

D-63

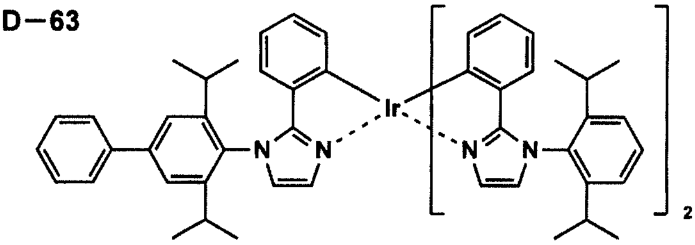

D-64

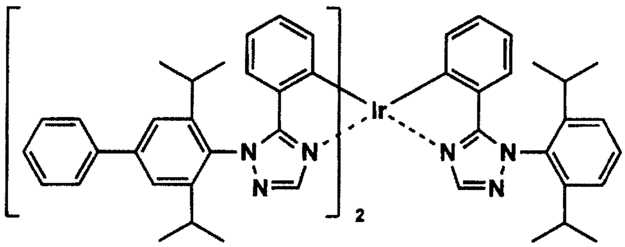

D-65

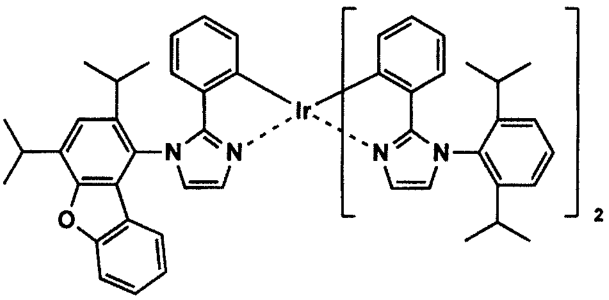

[Chem. 36]

D-66

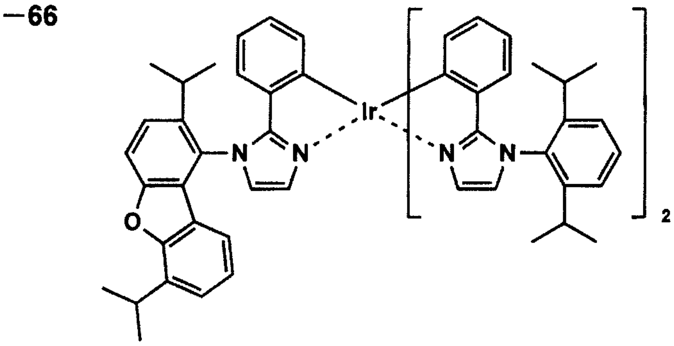

D-67

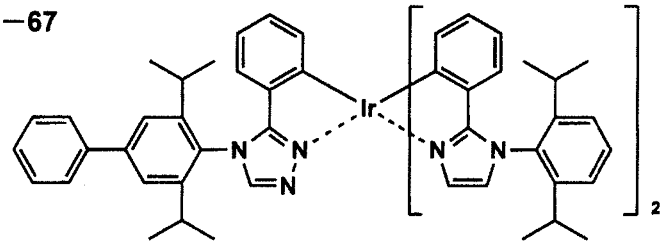

D-68

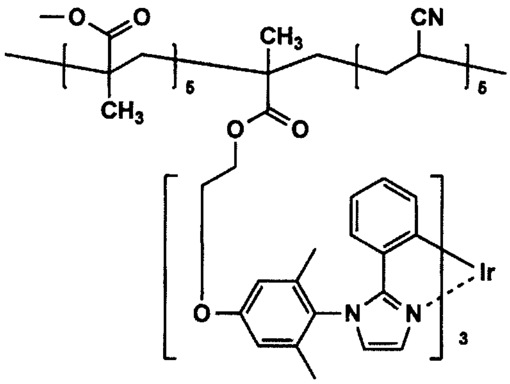

D-69

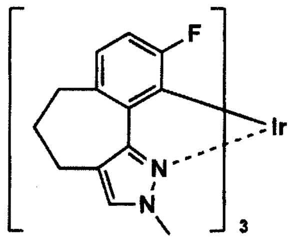

D-70

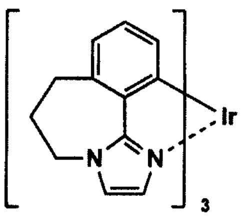

D-71

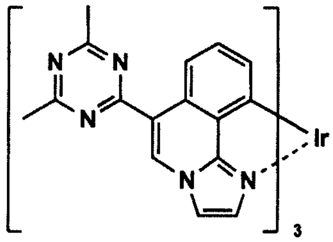

D-72

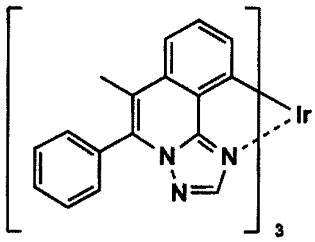

[Chem. 37]

D-73

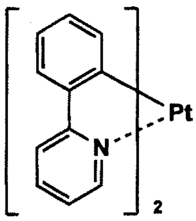

D-74

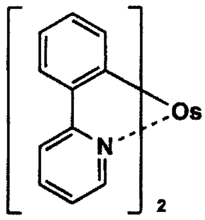

D-75

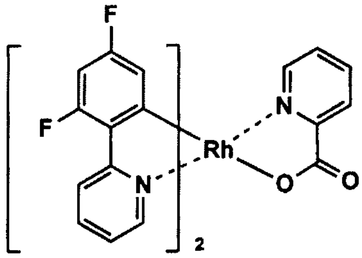

D-76

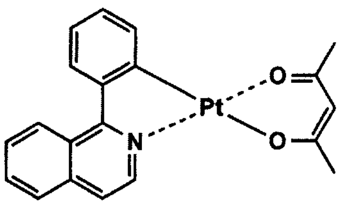

D-77

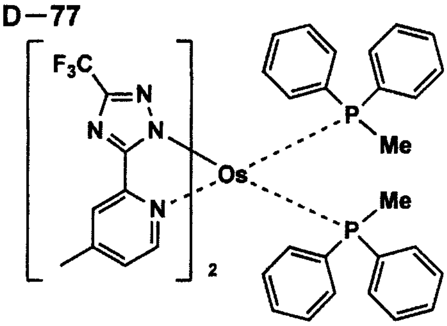

D-78

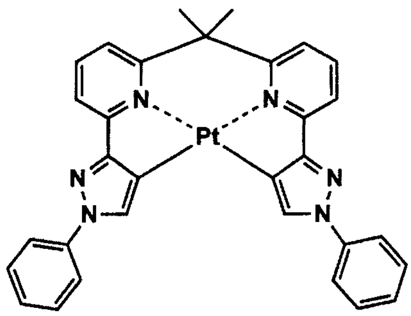

D-79

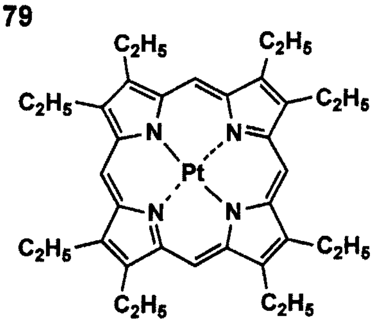

D-80

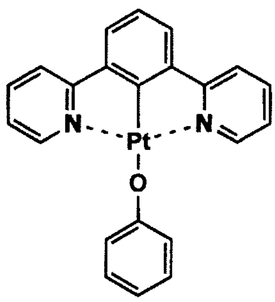

D-81

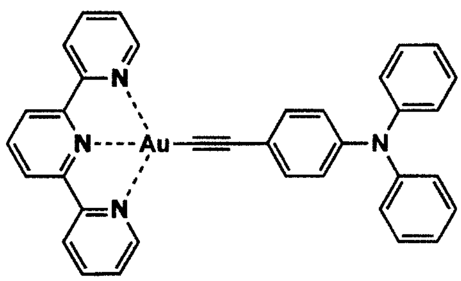

Of the known phosphorescent dopants usable in the present invention, DPs preferably used are (D-36), (D-37), (D-41), (D-53), (D-54), (D-55), (D-56), (D-61), (D-67) and (D-80), far preferably used are (D-41), (D-53), (D-54), (D-55) and (D-56), and still far preferably used are (D-53), (D-54) and (D-55).

(2) Host Compound

The host compound (also called a host material) of the present invention is a compound which mainly plays a role of injecting and transporting charges in the light emitting layer. In an organic EL element, light emission from the host compound itself is not observed substantially.

The host compound is a compound exhibiting, at room temperature (25°C), preferably a phosphorescent quantum yield of phosphorescence emission of less than 0.1, and far preferably a phosphorescent quantum yield thereof of less than 0.01. Further, of the compounds contained in the light emitting layer, a mass ratio of the host compound in the

layer is preferably 20% or more.

The exited state energy of the host compound is preferably higher than the exited state energy of the luminescent dopant contained in the same layer.

The host compounds may be used individually or multiple types thereof may be used together. Use of multiple types of host compounds enables adjustment of charge transfer, thereby increasing efficiency of an organic EL element.

The host compound usable in the present invention is not particularly limited to but may be a low molecular weight compound, a polymer having a repeating unit, or a compound having a reactive group such as a vinyl group or an epoxy group.

Of the known host compounds, a preferable one is a compound having a hole transporting ability or an electron transporting ability as well as preventing elongation of an emission wavelength and, in order to stably operate an organic EL element when the element is driven at high temperature or against heat generated while the element is being driven, having a high Tg (glass transition temperature) . Tg is preferably 90°C or more, and far preferably 120°C or more.

Here, the glass transition temperature (Tg) is a value obtained by using DCS (Differential Scanning Colorimetry) by a method in conformity with JIS-K-7121.

Specific examples of the known host compounds used in an organic EL element of the present invention include compounds described, for example, in the following documents, but the present invention is not limited thereto.

The documents include: Japanese Patent Application Publication Nos. 2002-203683, 2002-363227, 2002-234888, 2002-280183, 2002-299060, 2002-302516, 2002-305083 and 2002-305084; U.S. Patent Application Publication Nos. 2009/0017330, 2009/0030202 and 2005/0238919; International Patent Application Publication Nos. 2001/039234, 2009/021126, 2008/056746, 2007/063796, 2007/063754, 2004/107822, 2006/114966, 2009/086028, 2009/003898 and 2012/023947; Japanese Patent Application Publication Nos. 2008-074939 and 2007-254297; and EP 2034538.

«Electron Transport Layer»

The electron transport layer of the present invention is composed of a material having a function of transporting electrons and is only required to have a function of transmitting electrons injected from the cathode to the light emitting layer.

The thickness of the electron transport layer is not particularly limited, but generally in the range from 2 nm to 5 μm, preferably in the range from 2 nm to 500 nm, and far preferably in the range from 5 nm to 200 nm.

It is known that, in an organic EL element, when light produced in a light emitting layer is extracted from an electrode, light directly extracted from the light emitting layer interferes with light extracted after reflected by an electrode opposite the electrode from which the light is extracted. In the case where the light is reflected by the cathode, this interference effect can be efficiently utilized by appropriately adjusting the total thickness of the electron transport layer in the range from several nm to several μm.

Meanwhile, voltage is easily increased when the electron transport layer is made thick. Therefore, when the thickness is large especially, electron mobility in the electron transport layer is preferably $10^{-5}$ $cm^2/Vs$ or more.

As a material used for the electron transport layer (hereinafter called an electron transport material), it is only required to have either a property of injecting or transporting electrons or a barrier property against holes. Any of the conventionally known compounds can be selected and used.

Examples thereof include: a nitrogen-containing aromatic heterocycle derivative (a carbazole derivative, an azacarbazole derivative (formed such that one or more carbon atoms of a carbazole ring is substituted by a nitrogen atom (s)), a pyridine derivative, a pyrimidine derivative, a triazine derivative, a quinoline derivative, a quinoxaline derivative, a phenanthroline derivative, an oxazole derivative, a thiazole derivative, an oxadiazole derivative, a triazole derivative, a benzimidazole derivative, a benzoxazole derivative, etc.); a dibenzofuran derivative; a dibenzothiophene derivative; and an aromatic hydrocarbon ring derivative (a naphthalene derivative, an anthracene derivative, triphenylene, etc.).

Further, metal complexes each having a ligand of a quinolinol skeleton or a dibnenzoquinolinol skeleton such as tris(8-quinolinol)aluminum (Alq),
tris(5,7-dichloro-8-quinolinol)aluminum,
tris(5,7-dibromo-8-quinolinol)aluminum,
tris(2-methyl-8-quinolinol)aluminum,
tris(5-methyl-8-quinolinol)aluminum and
bis(8-quinolinol)zinc (Znq); and metal complexes each formed such that central metal of each of the above metal complexes is substituted by In, Mg, Cu, Ca, Sn, Ga or Pb can also be used as the electron transport material.

Still further, a phthalocyanine derivative and the distyrylpyrazine derivative, which is cited as an example of the material for the light emitting layer, can also be used as the electron transport material. Yet further, inorganic semiconductors can also be used as the electron transport material, as with the hole injection layer and the hole transport layer.

Polymer materials in each of which any of the above materials is introduced into a polymer chain or constitutes a main chain of a polymer can also be used.

As the electron transport layer of the present invention, the electron transport layer may be doped with a dope material as a guest material so as to form an (electron-rich) electron transport layer having high n property. Examples of the dope material include n type dopants, for example, metal compounds such as a metal complex and a metal halide. Specific examples of the electron transport layer having such a structure include those described in documents such as: Japanese Patent Application Publication Nos. 4-297076, 10-270172, 2000-196140 and 2001-102175; and J. Appl. Phys., 95, 5773 (2004).

Specific examples of the known electron transport materials preferably used in an organic EL element of the present invention include compounds described in the following documents, but the present invention is not limited thereto.

The documents include: U.S. Patent Application Publication Nos. 2009/0115316 and 2009/0179554; International Patent Application Publication Nos. 2003/060956 and 2008/132085; Appl. Phys. Lett. 75, 4 (1999) ; Appl. Phys. Lett. 81, 162 (2002); Appl. Phys. Lett. 81, 162 (2002); Appl. Phys. Lett. 79, 156 (2001); U.S. Patent Application Publication No. 2009/030202; International Patent Application Publication Nos. 2004/080975, 2005/085387, 2006/067931, 2007/086552, 2008/114690, 2009/069442, 2009/066779, 2009/054253, 2011/086935, 2010/150593 and 2010/047707; Japanese Patent Application Publication Nos. 2010-251675, 2009-209133, 2009-124114, 2008-277810, 2006-156445 and 2003-31367; and International Patent Application Publication No. 2012/115034.

Examples of the electron transport materials far preferably used in the present invention include: a pyridine derivative, a pyrimidine derivative, a pyrazine derivative, a triazine derivative, a dibenzofuran derivative, a dibenzothiophene derivative, a carbazole derivative, an azacarbazole derivative and a benzimidazole derivative.

The electron transport materials may be used individually or multiple types thereof may be used together.

«Hole Blocking Layer»

The hole blocking layer is a layer having a function of the electron transport layer in a broad sense. The hole blocking layer is preferably composed of a material having a function of transporting electrons with a small ability of transporting holes and can increase recombination probability of electrons and holes by blocking holes while transporting electrons.

The structure of the electron transport layer described above can be used for the hole block layer of the present invention as needed.

The hole blocking layer disposed in an organic EL element of the present invention is preferably disposed adjacent to the light emitting layer on the cathode side.

The thickness of the hole blocking layer of the present invention is preferably in the range from 3 nm to 100 nm, and far preferably in the range from 5 nm to 30 nm.

As the material used for the hole blocking layer, the above materials used for the electron transport layer are preferably used, and the above materials used as the host compound are also preferably used for the hole blocking layer.

«Electron Injection Layer»

The electron injection layer (also called a "cathode buffer layer") of the present invention is a layer disposed between the cathode and the light emitting layer for reduction in drive voltage and increase in emission luminance, which is detailed in Part 2, Chapter 2 "Denkyoku Zairyo (Electrode Material)" (pp. 123-166) of "Yuki EL Soshi To Sono Kogyoka Saizensen (Organic EL Element and Front of Industrialization thereof) (November 30, 1998, published by N.T.S Co., Ltd.)".

In the present invention, the electron injection layer may be provided according to necessity and may be present between the cathode and the light emitting layer or between the cathode and the electron transport layer.

The electron injection layer is preferably a very thin film. The thickness thereof is preferably in the range from 0.1 nm to 5 nm depending on the material thereof. The layer may be an uneven film in which the constituent material intermittently exists.

The electron injection layer is also detailed in documents such as Japanese Patent Application Publication Nos. 6-325871, 9-17574 and 10-74586, and specific examples of a material preferably used for the electron injection layer include: a metal (strontium, aluminum, etc.); an alkali metal compound (lithium fluoride, sodium fluoride, etc.); an alkali earth metal compound (magnesium fluoride, calcium fluoride, etc.); a metal oxide (aluminum oxide, etc.); and a metal complex (lithium 8-hydroxyquinolinato (Liq), etc.). The above electron transport materials may also be used therefor.

The above materials used for the electron injection layer may be used individually or multiple types thereof may be used together.

«Hole Transport Layer»

The hole transport layer of the present invention is composed of a material having a function of transporting holes and is only required to have a function of transmitting holes injected from the anode to the light emitting layer.
The thickness of the hole transport layer is not particularly limited, but generally in the range from 2 nm to 5 μm, preferably in the range from 5 nm to 500 nm, and far preferably in the range from 5 nm to 200 nm.
The material used for the hole transport layer (hereinafter called a hole transport material) is only required to have either a property of injecting or transporting holes or a barrier property against electrons. Any of the conventionally known compounds can be selected and used.
Examples thereof include: a porphyrin derivative; a phthalocyanine derivative; an oxazole derivative; a phenylenediamine derivative; a stilbene derivative; a triarylamine derivative; a carbazole derivative; an indolocarbazole derivative; an acene-based derivative such as anthracene and naphthalene; a fluorene derivative; a fluorenone derivative; polyvinyl carbazole; a polymer or oligomer in which aromatic amine is introduced to a main chain or a side chain; polysilane; and a conductive polymer or oligomer (e.g., PEDOT:PSS, an aniline-based copolymer, polyaniline, polythiophene, etc.).
Examples of the triarylamine derivative include: a benzidine type such as α-NPD, a star burst type such as MTDATA, and a compound having fluorenone or anthracene at a triarylamine linking core part.
Hexaazatriphenylene derivatives described in documents such as Japanese Patent Application Publication (Translation of PCT Application) No. 2003-519432 and Japanese Patent Application Publication No. 2006-135145 can also be used as the hole transport material.
The hole transport layer doped with impurities, thereby having high p property can also be used. Examples thereof include those described in documents such as Japanese Patent Application Publication Nos. 4-297076, 2000-196140 and 2001-102175, and J. Appl. Phys., 95, 5773 (2004).
It is also possible to use so-called p type hole transport materials described in documents such as Japanese Patent Application Publication No. 11-251067 and Appl. Phys. Lett. 80 (2002), p. 13 9 by J. Huang et al. , and inorganic compounds such as a p type-Si and a p type-SiC. Further, an ortho-metalated organic metal complex having Ir or Pt as central metal, such as Ir(ppy)3, is also preferably used.
Although the above ones can be used as the hole transport material, preferably used are a triarylamine derivative, a carbazole derivative, an indolocarbazole derivative, an organic metal complex, a polymer material or oligomer in which aromatic amine is introduced to a main chain or a side chain and the like.
Specific examples of the known hole transport materials preferably used in an organic EL element of the present invention also include compounds described in the following documents in addition to the above documents, but the present invention is not limited thereto.
The documents include: Appl. Phys. Lett. 69, 2160 (1996); Appl. Phys. Lett. 78, 673 (2001); Appl. Phys. Lett. 90, 183503 (2007); Appl. Phys. Lett. 51, 913 (1987); Synth. Met. 87, 171 (1997) ; Synth. Met. 91, 209 (1997) ; Synth. Met. 111, 421 (2000) ; SID Symposium Digest, 37, 923 (2006); U.S. Patent Application Publication Nos. 2003/0162053, 2006/0240279 and 2008/0220265; International Patent Application Publication Nos. 2007/002683 and 2009/018009; EP 650955; U.S. Patent Application Publication Nos. 2008/0124572, 2007/0278938, 2008/0106190 and 2008/0018221; International Patent Application Publication No. 2012/115034; Japanese Patent Application Publication (Translation of PCT Application) No. 2003-519432; and Japanese Patent Application Publication No. 2006-135145.
The hole transport materials may be used individually or multiple types thereof may be used together.

«Electron Blocking Layer»

The electron blocking layer is a layer having a function of the hole transport layer in a broad sense. The electron blocking layer is preferably composed of a material having a function of transporting holes with a small ability of transporting electrons and can increase recombination probability of electrons and holes by blocking electrons while transporting holes.
The structure of the hole transport layer described above can be used for the electron blocking layer of the present invention as needed.
The electron blocking layer disposed in an organic EL element of the present invention is preferably disposed adjacent to the light emitting layer on the anode side.
The thickness of the electron blocking layer of the present invention is preferably in the range from 3 nm to 100 nm, and far preferably in the range from 5 nm to 30 nm.
As the material used for the electron blocking layer, the above materials used for the hole transport layer are preferably used, and the above materials used as the host compound are also preferably used for the electron blocking layer.

«Hole Injection Layer»

The hole injection layer (also called an "anode buffer layer") of the present invention is a layer disposed between the anode and the light emitting layer for reduction in drive voltage and increase in emission luminance, which is detailed in Part 2, Chapter 2 "Denkyoku Zairyo (Electrode Material) " (pp. 123-166) of "Yuki EL Soshi To Sono Kogyoka Saizensen (Organic EL Element and Front of Industrialization thereof) (November 30, 1998, published by N.T.S Co., Ltd.)".
In the present invention, the hole injection layer may be provided according to necessity and, as described above, may be present between the anode and the light emitting layer or between the anode and the hole transport layer.
The hole injection layer is also detailed in documents such as Japanese Patent Application Publication Nos. 9-45479, 9-260062 and 8-288069, and examples of a material used for the hole injection layer include the above materials used for the hole transport layer.
Of these, preferable are phthalocyanine derivatives such as copper phthalocyanine; hexaazatriphenylene derivatives described in documents such as Japanese Patent Application Publication (Translation of PCT Application) No. 2003-519432 and Japanese Patent Application Publication No. 2006-135145; metal oxides such as vanadium oxide; amorphous carbon; conductive polymers such as polyaniline (emeraldine) and polythiophene; ortho-metalated complexes such as a tris(2-phenylpyridine)iridium complex; triarylamine derivatives; and the like.
The above materials used for the hole injection layer may be used individually or multiple types thereof may be used together.

«Light Collection Sheet»

An organic EL element of the present invention can increase luminance in a specific direction by collecting light in the specific direction, for example, in the front direction with respect to the luminescent surface of the element, through a process to provide, for example, a micro-lens array structure on the light extraction side of the support substrate (substrate) or through a light collection sheet combined.
In an example of the micro-lens array, square pyramids being a one side of 30 μm and an apex angle of 90 degrees are two-dimensionally arranged on the light extraction side of the substrate. The one side is preferably 10 μm to 100 μm. When it is less than the lower limit, coloration occurs due to generation of the diffraction effect, whereas when it exceeds the upper limit, the thickness increases undesirably.
As the light collection sheet, for example, those put into practical use in LED backlights of liquid crystal display devices can be used. As such sheets, for example, a brightness enhancement film (BEF), produced by Sumitomo 3M Limited, can be used. Examples of the shape of a prism sheet include Δ shaped stripes formed on a substrate, a shape in which the apex angle is rounded, a shape in which the pitch is randomly changed, and other shapes.
Further, in order to control an angle of radiation from the organic EL element, a light diffusion plate/film may be used together with the light collection sheet. For example, a diffusion film (LIGHT-UP), produced by Kimoto Co. , Ltd, can be used.

«Applications»

An organic EL element of the present invention can be used in display devices, displays and various types of light emitting sources.
Examples of the light emitting sources are not limited to but include: lighting devices (household lights and interior lights), backlights of timepieces and liquid crystal display devices, sign advertisements, signals, light sources of optical storage media, light sources of electrophotographic copiers, light sources of optical communication processors and light sources of optical sensors. The organic EL element can be effectively used, in particular, as a backlight of a liquid crystal display device and a light source of a lighting device.
Hereinafter, an example of a display device provided with organic EL elements of the present invention is described with reference to the drawings. FIG. 1 is a schematic view showing an example of a display device constituted of the organic EL elements. FIG. 1 is a schematic view of a display of a mobile phone or the like which displays image information by light emission of the organic EL elements.
A display 1 includes a display section A having a plurality of pixels and a control section B which performs image scanning on the display section A based on image information. The control section B is electrically connected to the display section A and sends scanning signals and image data signals to the pixels based on image information from the outside to make the pixels of each scanning line successively emit light in response to the scanning signal and according to the image data signal, thereby performing image scanning and displaying the image information on the display section A.
FIG. 2 is a schematic view of the display section A.
The display section A is provided with, on a substrate: a wiring section which contains a plurality of scanning

lines 5 and a plurality of data lines 6; and a plurality of pixels 3; and the like. The primary members of the display section A are described in the following.

In FIG. 2, light emitted by the pixels 3 is extracted along a white allow direction (downward direction).

The scanning lines 5 and the data lines 6 of the wiring section each are made of a conductive material, and the scanning lines 5 and the data lines 6 orthogonally intersect in a grid form and are connected to the pixels 3 at positions where the lines 5 and 6 orthogonally intersect (details are not shown in the drawing).

The pixels 3 receive image data signals from the data lines 6 when scanning signals are applied from the scanning lines 5 and emit light according to the received image data signals. Full-color display can be carried out with pixels having an emission color of a red region, pixels having an emission color of a green region and pixels having an emission color of a blue region appropriately apposed on the same substrate.

Next, an emission process of a pixel is described. FIG. 3 is a circuit diagram of a pixel. A pixel is provided with an organic EL element 10, a switching transistor 11, a driving transistor 12, a capacitor 13 and the like. Organic EL elements which emit red light, green light and blue light are used as organic EL elements 10 provided for pixels, and full-color display can be carried out with these apposed on the same substrate.

In FIG. 3, an image data signal is applied to the drain of the switching transistor 11 via the data line 6 from the control section B. Then, when a scanning signal is applied to the gate of the switching transistor 11 via the scanning line 5 from the control section B, the switching transistor 11 turns its drive on and transmits the image data signal, which is applied to the drain, to the capacitor 13 and the gate of the driving transistor 12.

By the image data signal being transmitted, the capacitor 13 is charged according to the potential of the image data signal, and also the driving transistor 12 turns its drive on. The drain of the driving transistor 12 is connected to a power source line 7 and the source of the driving transistor 12 is connected to the electrode (s) of the organic EL element 10, and an electric current is supplied from the power source line 7 to the organic EL element 10 according to the potential of the image data signal, which is applied to the gate of the driving transistor 12.

When the scanning signal is transferred to the next scanning line 5 by successive scanning by the control section B, the switching transistor 11 turns its drive off. However, because the capacitor 13 keeps the charged potential of the image data signal even if the switching transistor 11 turns its drive off, the driving transistor 12 keeps its drive in the on state, so that the organic EL element 10 keeps emitting light until the next scanning signal is applied. When the next scanning signal is applied by successive scanning, the driving transistor 12 turns its drive on according to the potential of the next image data signal which is synchronized with that scanning signal, so that the organic EL element 10 emits light.

That is, for the organic EL elements 10 of the pixels, switching transistors 11 and driving transistors 12 as active elements are provided, whereby the organic EL elements 10 of the pixels 3 emit light. This type of light emission method is called an active matrix system.

The organic EL elements 10 may emit light of multiple gradations based on a multiple-valued image data signal having multiple gradation potentials or may be on and off of a predetermined light emission quantity based on a binary image data signal. Further, the potential of the capacitor 13 may be kept until the next scanning signal is applied or may be discharged immediately before the next scanning signal is applied.

The present invention may employ, instead of the above-described active matrix system, a passive matrix system, by which organic EL elements emit light according to data signals only when scanning signals are scanned.

FIG. 4 is a schematic view of a display device employing the passive matrix system. In FIG. 4, scanning lines 5 and image data lines 6 are disposed in a grid form with pixels 3 interposed therebetween. When a scanning signal for scanning lines 5 is applied to a scanning line 5 by successive scanning, pixels 3 connected to the scanning line 5 emit light according to an image data signal. In the passive matrix system, pixels 3 are provided with no active elements, and therefore manufacturing costs can be reduced.

An organic EL element of the present invention may be subjected to patterning through a metal mask, by an inkjet printing method or the like as needed when layers are formed. In the case where patterning is carried out, only an electrode may be subjected to patterning, an electrode and a light emitting layer may be subjected to patterning, or all the layers of the element may be subjected to patterning, and in producing the element, conventional methods can be used therefor. «Embodiment of Lighting Device of Present Invention»

An embodiment of a lighting device provided with organic EL elements of the present invention is described.

The non-luminescent surface of each organic EL element of the present invention is covered with a glass cover. A 300 μm thick glass substrate is used as a sealing substrate, and an epoxy-based photo-curable adhesive (LUXTRAK LC0629B produced by Toagosei Co. , Ltd.) as a sealing material is applied to the periphery thereof. The resulting product is disposed over a cathode to be brought into close contact with a transparent support substrate. The adhesive is irradiated with UV light from the glass substrate side, thereby being cured, so that the organic EL element is sealed. Thus, the lighting device as shown in FIG. 5 and FIG. 6 can be formed.

FIG. 5 is a schematic view of a lighting device, wherein an organic EL element 101 of the present invention is covered with a glass cover 102 (incidentally, sealing by the glass cover is carried out in a globe box under nitrogen

atmosphere (under atmosphere of a high purity nitrogen gas having a purity of 99.999% or more) so that the organic EL element 101 is not brought into contact with air).

FIG. 6 is a cross-sectional view of the lighting device. In FIG. 6, 105 represents a cathode, 106 represents an organic EL layer, and 107 represents a transparent electrode. The interior of the glass cover 102 is filled with a nitrogen gas 108, and a water catching agent 109 is provided therein.

Examples

Hereinafter, the present invention is detailed with Examples. However, the present invention is not limited thereto. Note that "part(s)" or "%" used in Examples stands for "volume% (percent by volume)" unless otherwise specified.

[First Example]

« Production of Organic EL Element »

(1) Production of Organic EL Element 101

On a 50 mm × 50 mm × 0.7 mm (thickness) glass substrate, ITO (indium tin oxide) was deposited to be 150 nm thick and subjected to patterning to form an anode, and then the transparent substrate provided with this ITO transparent electrode was subjected to ultrasonic cleaning with isopropyl alcohol, dried with a dry nitrogen gas and subjected to UV ozone cleaning for five minutes. On this substrate, a solution of poly(3,4-ethylenedioxythiophene)-polystyrenesulfonate (abbr. PEDOT/PSS, Baytron P Al 4083, produced by Bayer) diluted with pure water was deposited by spin coating and thereafter dried at 140°C for one hour to form a 50 nm thick hole injection layer. This transparent substrate was fixed to a substrate holder of a commercially-available vacuum deposition device.

Vapor-deposition crucibles of the vacuum deposition device were filled with materials for the respective constituent layers at their respective optimum amounts to produce an element. The vapor-deposition crucibles used were made of a material for resistance heating, such as molybdenum or tungsten.

After the pressure was reduced to a vacuum of $1 \times 10^{-4}$ Pa, the vapor-deposition crucible having compound HT-1 therein was electrically heated, and compound HT-1 was deposited on the hole injection layer at a deposition rate of 0.1 nm/sec to form a 10 nm thick hole transport layer.

Next, comparative compound 1 as a host material and D-63 as a phosphorescent material were co-deposited at a deposition rate of 0.1 nm/sec to be 85 volume% and 15 volume%, respectively, to form a 30 nm thick light emitting layer.

Next, compound HB-1 was deposited at a deposition rate of 0.1 nm/sec to form a 5 nm thick hole blocking layer, and subsequently, compound Alq was deposited at a deposition rate of 0.1 nm/sec to form a 30 nm thick electron transport layer. Further, potassium fluoride was formed to be 2 nm thick and thereafter aluminum was deposited to be 100 nm thick to form a cathode.

To the non-luminescent surface of the element, a can-shaped glass cover was attached with UV curable resin under nitrogen atmosphere. Thus, the organic EL element 101 was produced.

The compounds used in Examples have chemical structural formulae shown below.

[Chem. 38]

Comparative Compound 1

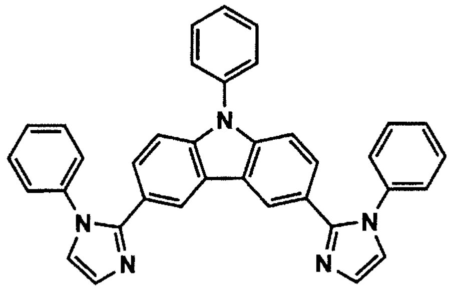

Comparative Compound 2

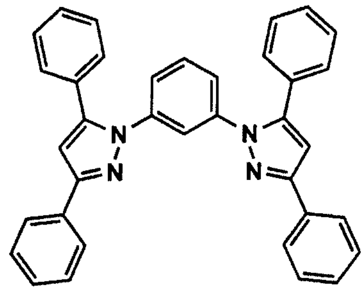

Comparative Compound 3

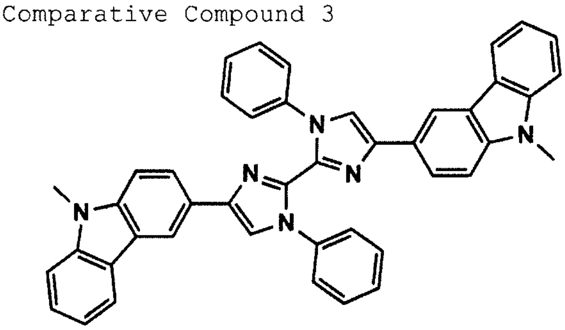

[Chem. 39]

HT-1

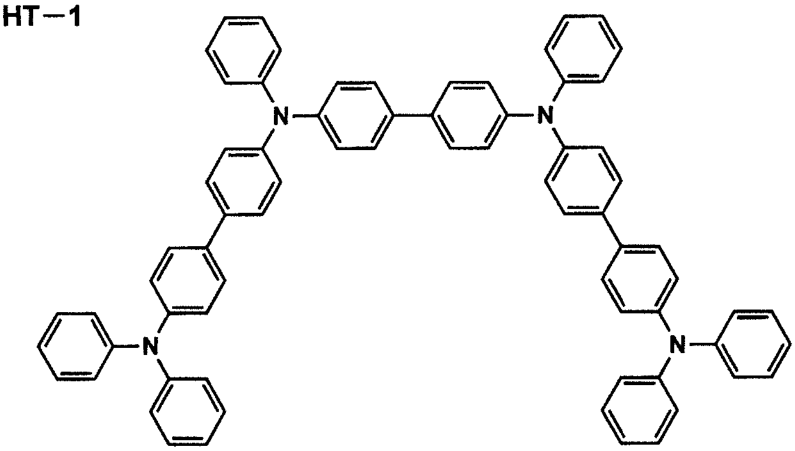

E-1

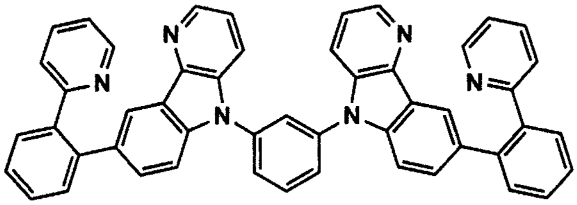

H-1

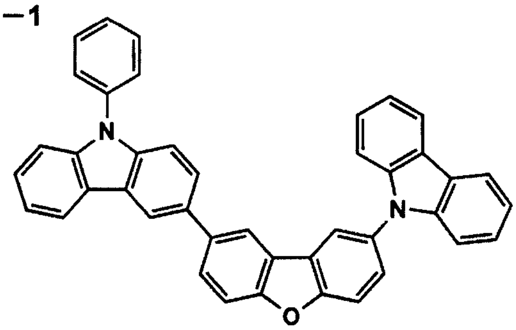

HB-1

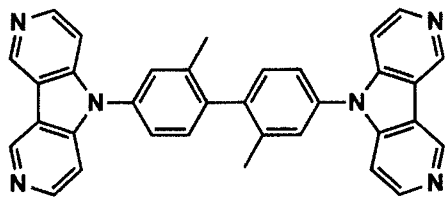

HAT

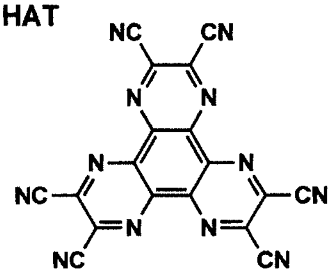

[Chem. 40]

D-4

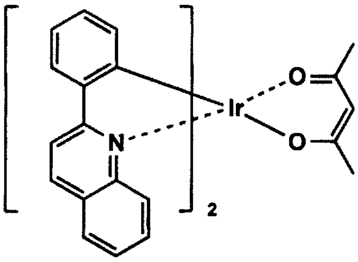

D-20

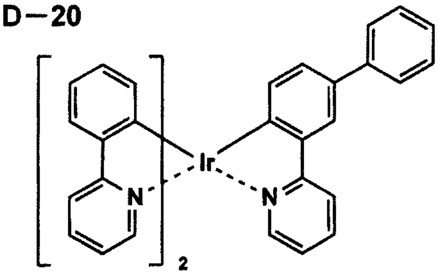

D-41

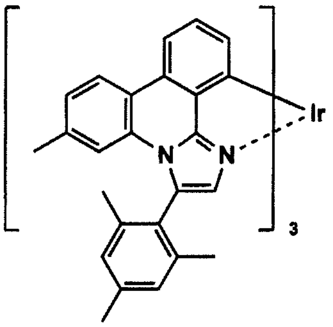

D-54

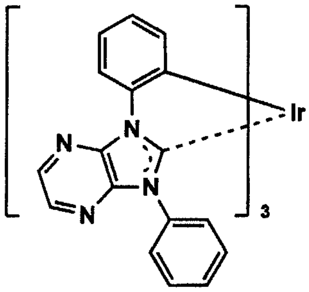

D-63

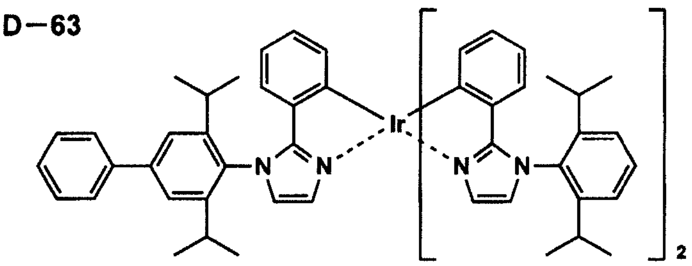

(2) Production of Organic EL Elements 102 to 120

The organic EL elements 102 to 120 were produced in the same way as the organic EL element 101 except that comparative compound 1 as the host compound was changed to the compounds shown in TABLE 1.

« Evaluation of Organic EL Elements 101 to 120»

With respect to the samples, the following evaluations were made. The evaluation result is shown in TABLE 1.

(1) Luminous Efficiency (EQE, External extraction Quantum Efficiency)

Each organic EL element was electrified at room temperature (about 23°C to 25°C) under a constant current condition of 2.5 $mA/cm^2$, and luminance (L0) [$cd/m^2$] immediately after start of light emission was measured, whereby the external extraction quantum efficiency ($\eta$) was calculated.

The luminance was measured with CS-2000 (produced by Konica Minolta Sensing Inc.). The external extraction quantum efficiency is shown by a relative value by taking that of the organic EL element 101 as 100.

The larger the value is, the more excellent the efficiency is.

(2) Half-life

In conformity with the following measurement method, half-life was evaluated.

Each organic EL element was driven with a constant current which provided initial luminance of 4,000 $cd/m^2$, time required for the luminance to be a half of the initial luminance was obtained, and this was used as the scale for the half-life. The half-life is shown by a relative value by taking that of the organic EL element 101 as 100.

The larger the value is, the more excellent the durability is.

(3) Exciton Stability

A part different from the element part used for the above (1) was irradiated by a UV-LED (5 W/$cm^2$) light source for 20 minutes. The distance between the light source and each sample at the time was 15 mm. After UV irradiation, a constant current of 2.5 mA/$cm^2$ was applied to each sample, luminance immediately after light emission was measured, a luminance residual ratio was calculated by using the following formula, and this was used as the scale for the exciton stability. The initial luminance was the luminance (L0) in the above (1) luminous efficiency evaluation.

Luminance Residual Ratio (%) = (Luminance after UV Irradiation for 20 min.) / (Initial Luminance (L0)) × 100

In TABLE 1, it is shown by a relative value by taking that of the organic EL element 101 as 100. The larger the value of the luminance residual ratio is, the more excellent the exciton stability is and the higher the durability of the organic EL element is.

(4) Heat Resistance

Each organic EL element was put in a constant temperature oven under a high temperature condition (about 50±5°C), half-life was evaluated by the same measurement method and condition as the above (2) half-life, and heat resistance was calculated by using the following formula.

Heat Resistance (%) = (Half-life under High Temperature Condition) / (Half-life at Room Temperature) × 100

In TABLE 1, it is shown by a relative value by taking that of the organic EL element 101 as 100. The higher the value of the heat resistance is, the higher the durability against temperature change is.

TABLE 1

| ELEMENT NO. | HOST MATERIAL | *1 | HALF-LIFE (RELATIVE VALUE) | EXCITON STABILITY (RELATIVE VALUE) | HEAT RESISTANCE (RELATIVE VALUE) | REMARK |
|---|---|---|---|---|---|---|
| 101 | COMPARATIVE COMPOUND 1 | 100 | 100 | 100 | 100 | *2 |
| 102 | COMPARATIVE COMPOUND 2 | 112 | 72 | 65 | 95 | *2 |
| 103 | COMPARATIVE COMPOUND 3 | 94 | 78 | 90 | 85 | *2 |
| 104 | (1 - 5) | 118 | 132 | 108 | 110 | *3 |
| 105 | (1 - 15) | 114 | 146 | 106 | 115 | *3 |
| 106 | (1 - 18) | 110 | 160 | 108 | 115 | *3 |
| 107 | (1 - 22) | 118 | 170 | 110 | 130 | *3 |
| 108 | (2 - 6) | 122 | 164 | 110 | 135 | *3 |
| 109 | (2 - 7) | 118 | 188 | 118 | 140 | *3 |
| 110 | (2 - 9) | 118 | 186 | 126 | 180 | *3 |
| 111 | (2 - 12) | 124 | 194 | 122 | 150 | *3 |
| 112 | (2 - 15) | 130 | 190 | 126 | 145 | *3 |

(continued)

| ELEMENT NO. | HOST MATERIAL | *1 | HALF-LIFE (RELATIVE VALUE) | EXCITON STABILITY (RELATIVE VALUE) | HEAT RESISTANCE (RELATIVE VALUE) | REMARK |
|---|---|---|---|---|---|---|
| 113 | (2 - 18) | 126 | 108 | 134 | 180 | *3 |
| 114 | (2 - 20) | 128 | 204 | 130 | 155 | *3 |
| 115 | (2 - 25) | 124 | 228 | 136 | 200 | *3 |
| 116 | (2 - 29) | 132 | 184 | 130 | 170 | *3 |
| 117 | (3 - 5) | 124 | 192 | 142 | 175 | *3 |
| 118 | (3 - 10) | 130 | 210 | 144 | 180 | *3 |
| 119 | (3 - 19) | 128 | 220 | 148 | 165 | *3 |
| 120 | (3 - 28) | 122 | 174 | 134 | 170 | *3 |
| *1 : LUMINOUS EFFICIENCY (RELATIVE VALUE)<br>*2 : COMPARATIVE EXAMPLE *3 : PRESENT INVENTION | | | | | | |

As it is understood from the above, when the compound of the present invention is used, the luminous efficiency is higher and the half-life is longer than when the comparative compound is used. Thus, the organic EL element using the compound of the present invention can have both high luminous efficiency and durability.

[Second Example]

(1) Production of Organic EL Element 201

The organic EL element 201 was produced in the same way as the organic EL element 101 except that the light emitting layer was formed such that comparative compound 1 as the host material and D-41 as the phosphorescent material were co-deposited at a deposition rate of 0.1 nm/sec to be 90 volume% and 10 volume%, respectively, to be 30 nm thick.

(2) Production of Organic EL Elements 202 to 215

The organic EL elements 202 to 215 were produced in the same way as the organic EL element 201 except that comparative compound 1 as the host compound was changed to the compounds shown in TABLE 2.

«Evaluation of Organic EL Elements 201 to 215»

With respect to the samples, the same evaluations made with respect to the organic EL elements 101 to 120 were made. The evaluation result is shown in TABLE 2.

TABLE 2

| ELEMENT NO. | HOST MATERIAL | *1 | HALF-LIFE (RELATIVE VALUE) | EXCITON STABILITY (RELATIVE VALUE) | HEAT RESISTANCE (RELATIVE VALUE) | REMARK |
|---|---|---|---|---|---|---|
| 201 | COMPARATIVE COMPOUND 1 | 100 | 100 | 100 | 100 | *2 |
| 202 | COMPARATIVE COMPOUND 2 | 122 | 108 | 84 | 76 | *2 |
| 203 | COMPARATIVE COMPOUND 3 | 92 | 102 | 80 | 88 | *2 |

(continued)

| ELEMENT NO. | HOST MATERIAL | *1 | HALF-LIFE (RELATIVE VALUE) | EXCITON STABILITY (RELATIVE VALUE) | HEAT RESISTANCE (RELATIVE VALUE) | REMARK |
|---|---|---|---|---|---|---|
| 204 | (1 - 9) | 124 | 130 | 104 | 114 | *3 |
| 205 | (1 - 12) | 118 | 136 | 106 | 110 | *3 |
| 206 | (1 - 17) | 120 | 144 | 110 | 116 | *3 |
| 207 | (1 - 28) | 124 | 142 | 110 | 116 | *3 |
| 208 | (2 - 6) | 126 | 154 | 116 | 120 | *3 |
| 209 | (2 - 9) | 118 | 160 | 122 | 126 | *3 |
| 210 | (2 - 17) | 120 | 168 | 124 | 124 | *3 |
| 211 | (2 - 20) | 128 | 180 | 126 | 126 | *3 |
| 212 | (2 - 25) | 126 | 184 | 130 | 136 | *3 |
| 213 | (2 - 28) | 124 | 176 | 128 | 134 | *3 |
| 214 | (3 - 5) | 130 | 186 | 134 | 128 | *3 |
| 215 | (3 - 22) | 124 | 168 | 124 | 130 | *3 |
| *1 : LUMINOUS EFFICIENCY (RELATIVE VALUE)<br>*2 : COMPARATIVE EXAMPLE *3 : PRESENT INVENTION | | | | | | |

As it is understood from the above, even if D-41 is used as the phosphorescent material, when the compound of the present invention is used as the host material, the luminous efficiency is higher and the half-life is longer than when the comparative compound is used as the host material. Thus, the organic EL element using the compound of the present invention can have both high luminous efficiency and durability.

[Third Example]

(1) Production of Organic EL Element 301

The organic EL element 301 was produced in the same way as the organic EL element 101 except that the light emitting layer was formed such that comparative compound 1 as the host material and D-54 as the phosphorescent material became 94 volume% and 6 volume%, respectively, to be 30 nm thick, and the hole blocking layer was formed by changing the hole blocking material from HB-1 to BAlq (bis(2-methyl-8-quinolinolato)(4-phenylphenolato)aluminum (III)).

(2) Production of Organic EL Elements 302 to 317

The organic EL elements 302 to 317 were produced in the same way as the organic EL element 301 except that comparative compound 1 as the host compound was changed to the compounds shown in TABLE 3.

«Evaluation of Organic EL Elements 301 to 317» With respect to the samples, the same evaluations made with respect to the organic EL elements 101 to 120 were made. The evaluation result is shown in TABLE 3.

TABLE 3

| ELEMENT NO. | HOST MATERIAL | *1 | HALF-LIFE (RELATIVE VALUE) | EXCITON STABILITY (RELATIVE VALUE) | HEAT RESISTANCE (RELATIVE VALUE) | REMARK |
|---|---|---|---|---|---|---|
| 301 | COMPARATIVE COMPOUND 1 | 100 | 100 | 100 | 100 | *2 |

(continued)

| ELEMENT NO. | HOST MATERIAL | *1 | HALF-LIFE (RELATIVE VALUE) | EXCITON STABILITY (RELATIVE VALUE) | HEAT RESISTANCE (RELATIVE VALUE) | REMARK |
|---|---|---|---|---|---|---|
| 302 | COMPARATIVE COMPOUND 2 | 107 | 66 | 58 | 84 | *2 |
| 303 | COMPARATIVE COMPOUND 3 | 82 | 68 | 102 | 92 | *2 |
| 304 | (1 - 15) | 112 | 120 | 108 | 108 | *3 |
| 305 | (1 - 17) | 128 | 136 | 124 | 114 | *3 |
| 306 | (1 - 24) | 130 | 140 | 138 | 110 | *3 |
| 307 | (2 - 6) | 130 | 138 | 128 | 112 | *3 |
| 308 | (2 - 7) | 134 | 146 | 132 | 122 | *3 |
| 309 | (2 - 9) | 132 | 152 | 136 | 126 | *3 |
| 310 | (2 - 17) | 134 | 150 | 138 | 118 | *3 |
| 311 | (2 - 20) | 136 | 156 | 140 | 124 | *3 |
| 312 | (2 - 25) | 138 | 162 | 144 | 142 | *3 |
| 313 | (2 - 30) | 138 | 158 | 138 | 132 | *3 |
| 314 | (2 - 33) | 136 | 160 | 142 | 134 | *3 |
| 315 | (2 - 38) | 132 | 154 | 144 | 130 | *3 |
| 316 | (3 - 9) | 140 | 166 | 140 | 140 | *3 |
| 317 | (3 - 25) | 134 | 158 | 134 | 130 | *3 |
| *1 : LUMINOUS EFFICIENCY (RELATIVE VALUE)<br>*2 : COMPARATIVE EXAMPLE *3 : PRESENT INVENTION | | | | | | |

As it is understood from the above, even if D-54 is used as the phosphorescent material, when the compound of the present invention is used as the host material, the luminous efficiency is higher and the half-life is longer than when the comparative compound is used as the host material. Thus, the organic EL element using the compound of the present invention can have both high luminous efficiency and durability.

[Forth Example]

(1) Production of Organic EL Element 401

The organic EL element 401 was produced in the same way as the organic EL element 115 except that comparative compound 1 was deposited on the hole transport layer at a deposition rate of 0.1 nm/sec to form a 10 nm second hole transport layer between the hole transport layer and the light emitting layer.

(2) Production of Organic EL Elements 402 to 410

The organic EL elements 402 to 410 were produced in the same way as the organic EL element 401 except that the material of the second hole transport layer was changed from comparative compound 1 to the compounds shown in TABLE 4.

«Evaluation of Organic EL Elements 401 to 410»

With respect to the samples, an evaluation of drive voltage was made in addition to the evaluation of the half-life made with respect to the organic EL elements 101 to 120. The evaluation result is shown in TABLE 4.

(1) Drive Voltage

The drive voltage applied when each organic EL element was electrified at room temperature (about 23°C to 25°C) under a constant current condition of 2.5 mA/cm$^2$ was measured. It is shown by a relative value by taking that of the organic EL element 401 as 100.
The smaller the value is, the lower the drive voltage is and the more excellent the luminous efficiency is.

(2) Half-life

The half-life was evaluated in the same way as the half-life was evaluated in First Example. The half-life is shown by a relative value by taking that of the organic EL element 401 as 100.
The larger the value is, the more excellent the durability is.

TABLE 4

| ELEMENT NO. | SECOND HOLE TRANSPORT MATERIAL | DRIVE VOLTAGE (RELATIVE VALUE) | HALF-LIFE (RELATIVE VALUE) | REMARK |
|---|---|---|---|---|
| 401 | COMPARATIVE COMPOUND 1 | 100 | 100 | COMPARATIVE EXAMPLE |
| 402 | COMPARATIVE COMPOUND 2 | 102 | 82 | COMPARATIVE EXAMPLE |
| 403 | COMPARATIVE COMPOUND 3 | 114 | 102 | COMPARATIVE EXAMPLE |
| 404 | (1 - 28) | 84 | 112 | PRESENT INVENTION |
| 405 | (2 - 6) | 80 | 118 | PRESENT INVENTION |
| 406 | (2 - 16) | 76 | 122 | PRESENT INVENTION |
| 407 | (2 - 24) | 78 | 126 | PRESENT INVENTION |
| 408 | (3 - 2) | 74 | 132 | PRESENT INVENTION |
| 409 | (3 - 19) | 72 | 138 | PRESENT INVENTION |
| 410 | (3 - 31) | 76 | 128 | PRESENT INVENTION |

As it is understood from the above, when the compound of the present invention is used as the hole transport material, driving can be carried out with a lower voltage and the half-life is longer than when the comparative compound is used as the hole transport material. Thus, the organic EL element using the compound of the present invention can have both low voltage driving and durability.

[Fifth Example]

« Production of White Organic EL Element »

(1) Production of Organic EL Element 501

On a 50 mm × 50 mm × 0.7 mm (thickness) glass substrate, ITO (indium tin oxide) was deposited to be 150 nm thick and subjected to patterning to form an anode, and then the transparent substrate provided with this ITO transparent electrode was subjected to ultrasonic cleaning with isopropyl alcohol, dried with a dry nitrogen gas and subjected to UV ozone cleaning for five minutes. Thereafter, this transparent substrate was fixed to a substrate holder

of a vacuum deposition device.

Vapor-deposition crucibles of the vacuum deposition device were filled with materials for the respective constituent layers at their respective optimum amounts to produce an element. The vapor-deposition crucibles used were made of a material for resistance heating, such as molybdenum or tungsten.

After the pressure was reduced to a vacuum of $1 \times 10^{-4}$ Pa, the vapor-deposition crucible having compound HAT therein was electrically heated, and compound HT-1 was deposited on the ITO transparent electrode at a deposition rate of 0.1 nm/sec to form a 15 nm thick hole injection layer.

Next, compound HT-1 was deposited in the same way to form a 70 nm thick hole transport layer.

Next, compound H-1, compound D-20 and compound D-4 were co-deposited at a deposition rate of 0.1 nm/sec to be 88 volume%, 10 volume% and 2 volume%, respectively, to form a 15 nm thick first light emitting layer.

Next, compound (2-25) of the present invention and compound D-63 were co-deposited at a deposition rate of 0.1 nm/sec to be 90 volume% and 15 volume%, respectively, to form a 20 nm thick second light emitting layer.

Next, compound HB-1 was deposited at a deposition rate of 0.1 nm/sec to form a 5 nm thick hole blocking layer. Thereafter, compound E-1 was deposited at a deposition rate of 0.1 nm/sec to form a 45 nm thick electron transport layer. Further, potassium fluoride was formed to be 2.0 nm thick and thereafter aluminum was deposited to be 100 nm thick to form a cathode.

The non-luminescent surface of the element was covered with a can-shaped glass cover and an electrode extraction wiring substrate was set under atmosphere of a high purity nitrogen gas having a purity of 99.999% or more. Thus, the organic EL element 501 was produced.

A lighting device as shown in FIG. 5 and FIG. 6 was produced by using the organic EL element (s) 501 and electrified. Then, white light was emitted. Thus, the organic EL elements each using the compound of the present invention can be used in a lighting device.

[Sixth Example]

<<Production of Full-color Organic EL Display Device>>

FIG. 7A to FIG. 7E are schematic configuration views of a full-color organic EL display device.

On a glass substrate 201, a substrate (NA45 produced by NH Techno Glass Co., Ltd) provided with an ITO transparent electrode (s) 202 formed to be 100 nm was subjected to patterning with a pitch of 100 μm to form anodes (FIG. 7A), and thereafter, between the ITO electrodes 202 on the glass substrate 201, non-photosensitive polyimide walls 203 (a width of 20 μm and a thickness of 2.0 μm) were formed by photolithography (FIG. 7B).

Onto the ITO electrodes 202 between the walls 203, a hole injection layer composite having the following composition was discharged/injected by using an inkjet head (MJ800C produced by Seiko Epson Corporation), irradiated with UV light for 200 seconds and dried at 60°C for 10 minutes to form 40 nm thick hole injection layers 204 (FIG. 7C).

Onto this hole injection layers 204 , a blue light emitting layer composite, a green light emitting layer composite and a red light emitting layer composite having the following respective compositions were discharged/injected in the same way by using the inkjet head and dried at 60°C for 10 minutes to form light emitting layers 205B, 205G and 205R of the respective colors (FIG. 7D).

(Hole Injection Layer Composite)
HT-1: 20 parts by mass
Cyclohexylbenzene: 50 parts by mass
Isopropylbiphenyl: 50 parts by mass
(Blue Light Emitting Layer Composite)
Compound (2-20) of Present Invention: 0.8 parts by mass
DP-55: 0.04 parts by mass
Cyclohexylbenzene: 50 parts by mass
Isopropylbiphenyl: 50 parts by mass
(Green Light Emitting Layer Composite)
H-1: 0.7 parts by mass
D-20: 0.04 parts by mass
Cyclohexylbenzene: 50 parts by mass
Isopropylbiphenyl: 50 parts by mass
(Red Light Emitting Layer Composite)
H-1: 0.7 parts by mass
D-4: 0.04 parts by mass
Cyclohexylbenzene: 50 parts by mass

Isopropylbiphenyl: 50 parts by mass

Next, to cover the light emitting layers 205B, 205G and 205R, an electron transport material (compound E-1) was deposited to form a 45 nm thick electron transport layer (s) (not shown), further lithium fluoride was deposited to form a 0.5 nm thick electron injection layer(s) (not shown), and Al was deposited to form a 130 nm thick cathode 206. Thus, organic EL elements were produced (FIG. 7E).

The produced organic EL elements emitted blue light, green light and red light with voltage applied to the electrodes. Thus, the organic EL elements can be used in a full-color display device.

As described above, according to the present invention, an organic electroluminescent element, a lighting device and a display device each having high luminous efficiency and excellent durability can be provided.

Further, by a wet process, the organic EL element having the above effects can be produced.

Industrial Applicability

A material for organic electroluminescent elements of the present invention has high triplet excitation energy and can provide an organic electroluminescent element, a lighting device and a display device, each using the material for organic electroluminescent elements, having high luminous efficiency and excellent durability.

Description of Reference Numerals

| | |
|---|---|
| 1 | Display |
| 3 | Pixel |
| 5 | Scanning Line |
| 6 | Data Line |
| 7 | Power Source Line |
| 10 | Organic EL Element |
| 11 | Switching Transistor |
| 12 | Driving Transistor |
| 13 | Capacitor |
| 101 | Organic EL Element |
| 102 | Glass Cover |
| 105 | Cathode |
| 106 | Organic EL Layer |
| 107 | Transparent Electrode |
| 108 | Nitrogen Gas |
| 109 | Water Catching Agent |
| 201 | Glass Substrate |
| 202 | Transparent Electrode |
| 203 | Wall |
| 204 | Hole Injection Layer |
| 205B, 205G, 205R | Light Emitting Layer of Each Color |
| A | Display Section |
| B | Control Section |
| L | Light |

**Claims**

**1.** A material for organic electroluminescent elements comprising a structure represented by the following General Formula (1):

[Chem. 1]

General Formula (1)

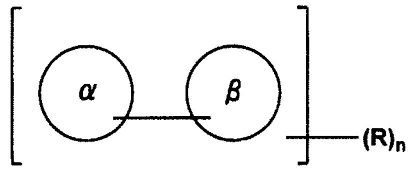

wherein a ring α and a ring β respectively represent aromatic heterocyclic groups each derived from pyrrole, furan, thiophene, pyrazole, 1,2,3-triazole, 1,2,4-triazole, tetrazole, oxazole, isoxazole, oxadiazole, thiazole, isothiazole or thiadiazole, and are linked with each other through arbitrary positions; R represents a hydrogen atom or a substituent substituted at an arbitrary position of at least one of the ring α and the ring β; and n represents an integer of 1 to 8.

2. The material for organic electroluminescent elements according to claim 1, wherein the structure represented by the General Formula (1) is a structure represented by the following General Formula (2):

[Chem. 2]

General Formula (2)

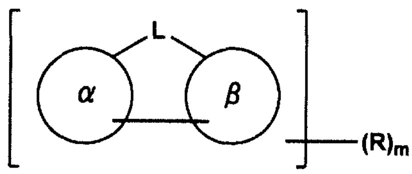

wherein a ring α, a ring β and R are synonymous with the ring α, ring β and R in the General Formula (1), respectively; m represents an integer of 1 to 6; and L represents a divalent linking group.

3. The material for organic electroluminescent elements according to claim 1, wherein the structure represented by the General Formula (1) is a structure represented by the following General Formula (1-1):

[Chem. 3]

General Formula (1-1)

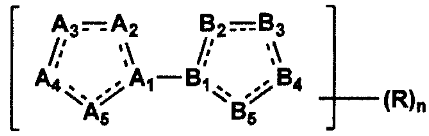

wherein $A_1$, $A_2$, $A_3$, $A_4$, $A_5$, $B_1$, $B_2$, $B_3$, $B_4$ and $B_5$ each represent a carbon atom, a nitrogen atom, an oxygen atom or a sulfur atom, and $A_1$ to $A_5$ and $B_1$ to $B_5$ respectively form 5-membered aromatic heterocycles each derived from pyrrole, furan, thiophene, pyrazole, 1,2,3-triazole, 1,2,4-triazole, tetrazole, oxazole, isoxazole, oxadiazole, thiazole,

isothiazole or thiadiazole; R represents a hydrogen atom or a substituent substituted at an arbitrary position of at least one of the two aromatic heterocyclic groups; and n represents an integer of 1 to 8.

**4.** The material for organic electroluminescent elements according to claim 2, wherein the structure represented by the General Formula (2) is a structure represented by the following General Formula (2-1):

[Chem. 4]

General Formula (2-1)

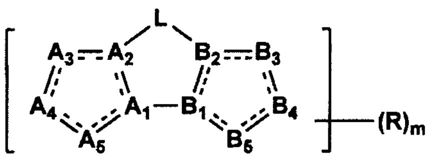

wherein $A_1$, $A_2$, $A_3$, $A_4$, $A_5$, $B_1$, $B_2$, $B_3$, $B_4$ and $B_5$ each represent a carbon atom, a nitrogen atom, an oxygen atom or a sulfur atom, and $A_1$ to $A_5$ and $B_1$ to $B_5$ respectively form 5-membered aromatic heterocycles each derived from pyrrole, furan, thiophene, pyrazole, 1,2,3-triazole, 1,2,4-triazole, tetrazole, oxazole, isoxazole, thiazole or isothiazole; R represents a hydrogen atom or a substituent substituted at an arbitrary position of at least one of the two aromatic heterocyclic groups; m represents an integer of 1 to 8; and L represents a divalent linking group.

**5.** An organic electroluminescent element comprising an organic layer including at least a light emitting layer interposed between an anode and a cathode, wherein
any of the organic layer contains the material for organic electroluminescent elements according to any one of claims 1 to 4.

**6.** The organic electroluminescent element according to claim 5, wherein the light emitting layer contains a phosphorescent compound.

**7.** The organic electroluminescent element according to claim 6, wherein the phosphorescent compound has a structure represented by the following General Formula (DP):

[Chem. 5]

General Formula (DP)

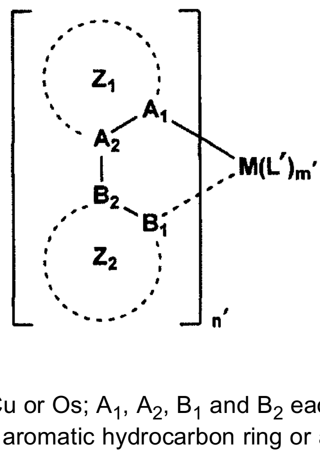

wherein M represents Ir, Pt, Rh, Ru, Ag, Cu or Os; $A_1$, $A_2$, $B_1$ and $B_2$ each represent a carbon atom or a nitrogen atom; a ring $Z_1$ represents a 6-membered aromatic hydrocarbon ring or a 5-or 6-membered aromatic heterocycle

formed with $A_1$ and $A_2$; a ring $Z_2$ represents a 5- or 6-membered aromatic heterocycle formed with $B_1$ and $B_2$; the ring $Z_1$ and the ring $Z_2$ may respectively have substituents, the substituents may be bonded with each other to form a condensed ring structure, and the substituents of ligands may be bonded with each other so that the ligands are linked with each other; L' represents a monoanionic bidentate ligand coordinated to M; m' represents an integer of 0 to 2, and n' represents an integer of 1 to 3, provided that m' + n' is 2 or 3; and when n' is 2 or more, the ligands represented by the ring $Z_1$ and the ring $Z_2$ may be the same or different from each other, and when m' is 2 or more, L's may be the same or different from each other.

**8.** A display device comprising the organic electroluminescent element according to any one of claims 5 to 7.

**9.** A lighting device comprising the organic electroluminescent element according to any one of claims 5 to 7.

# FIG.1

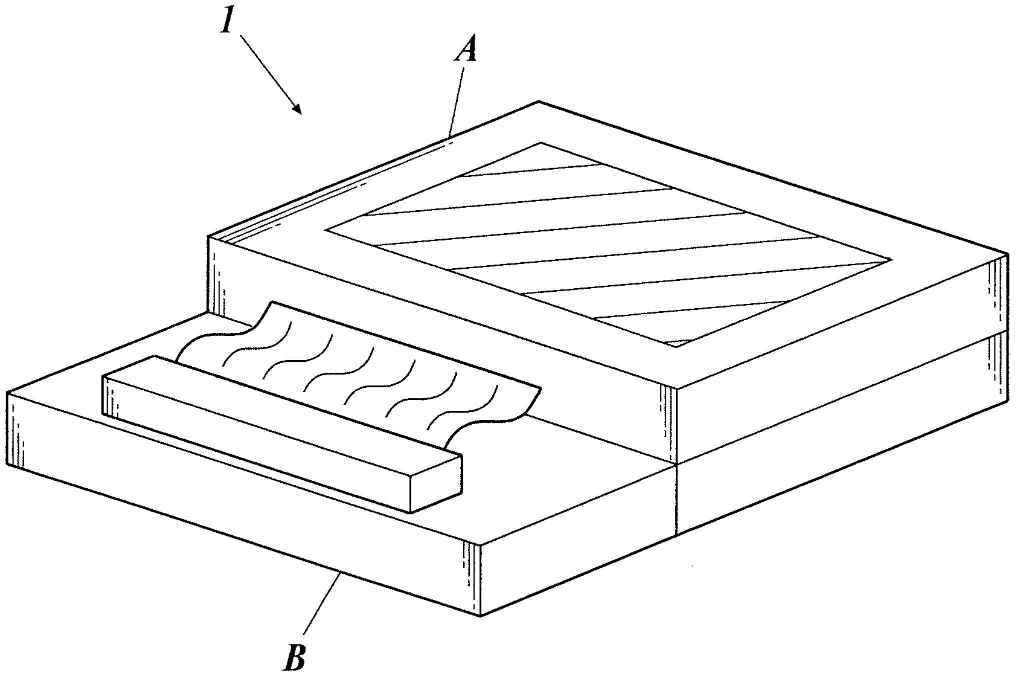


# FIG.2

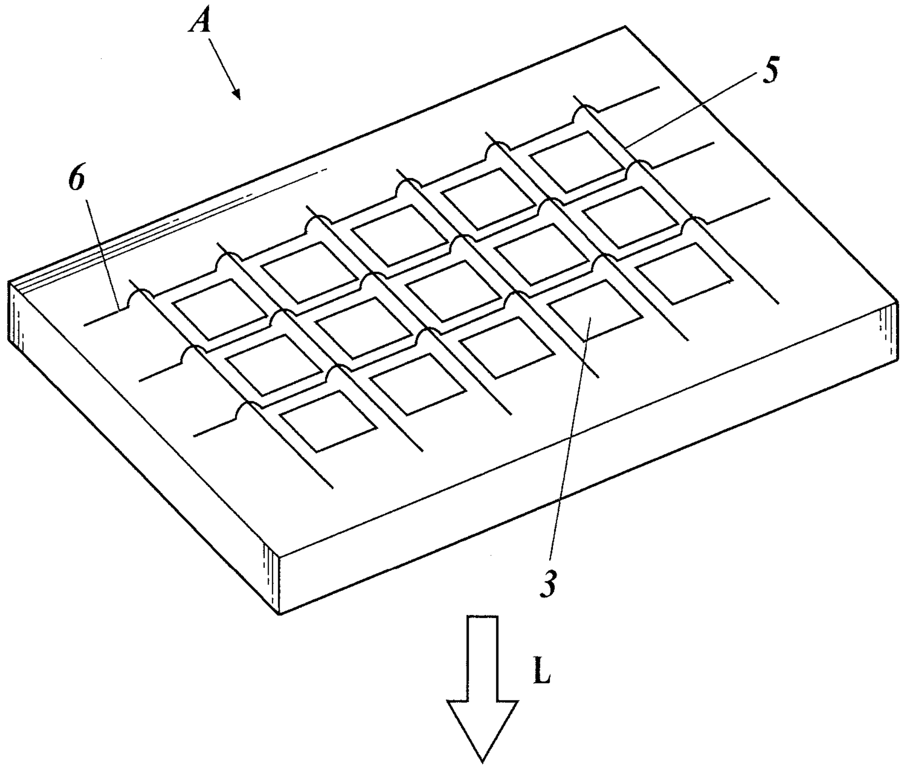

FIG.3

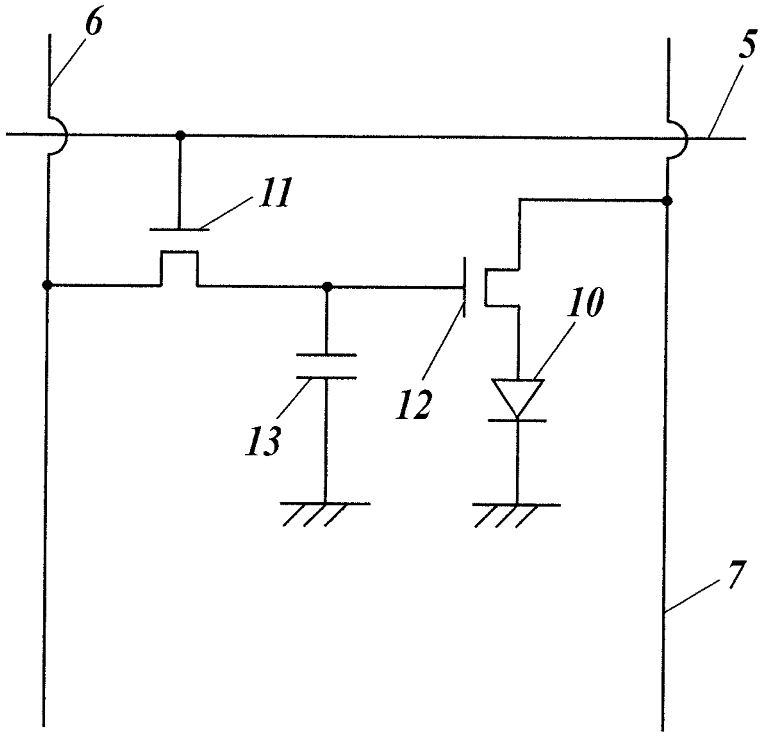

FIG.4

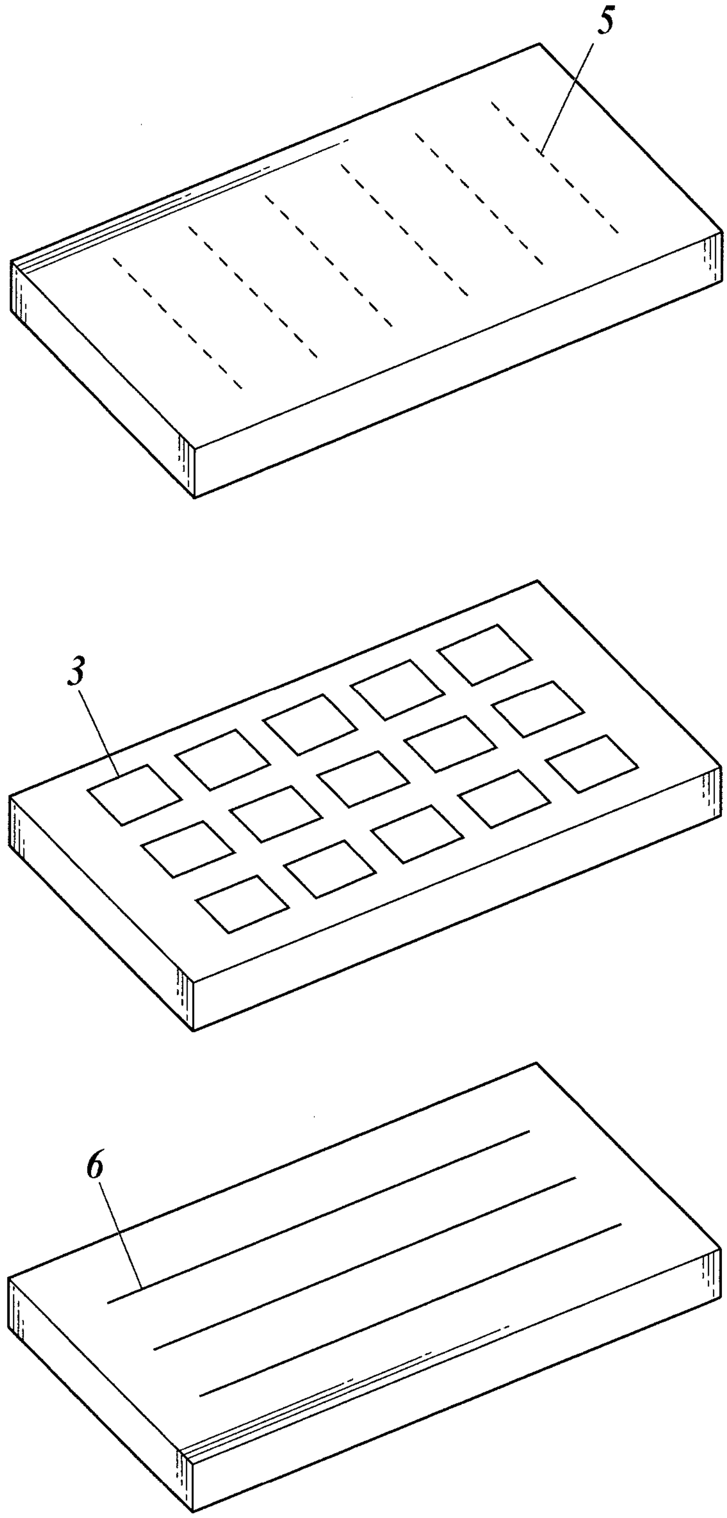

FIG.5

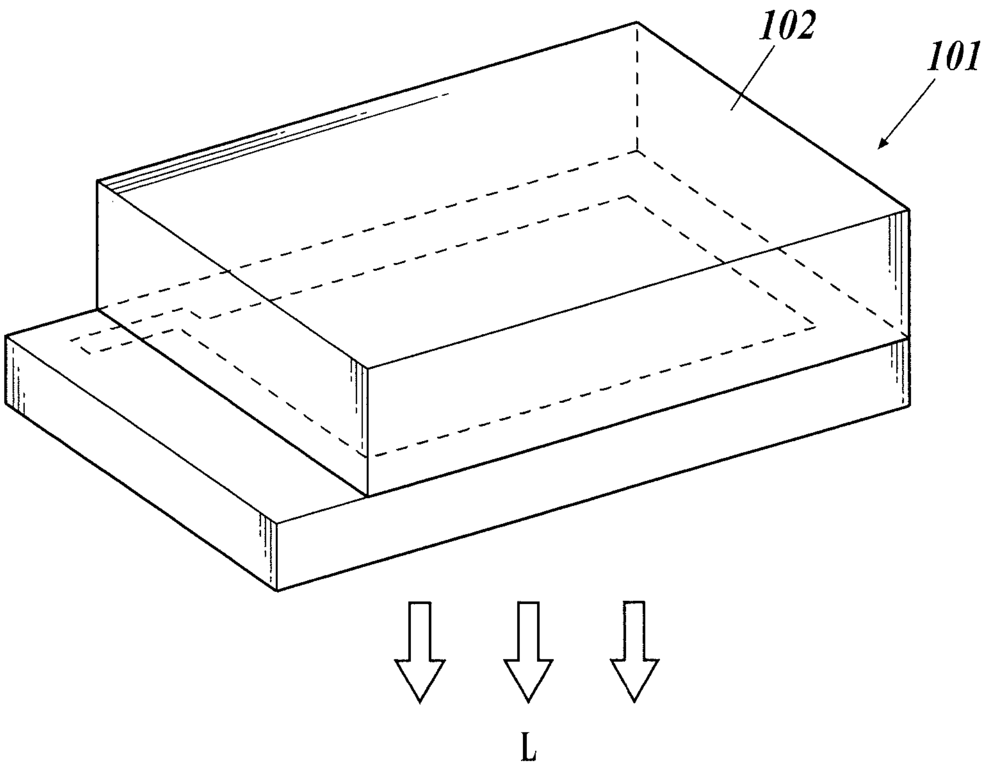


FIG.6

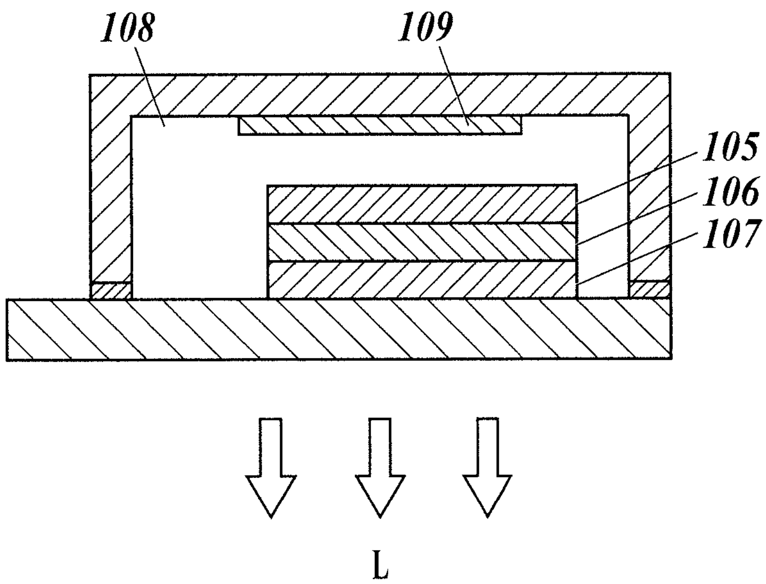

FIG. 7A

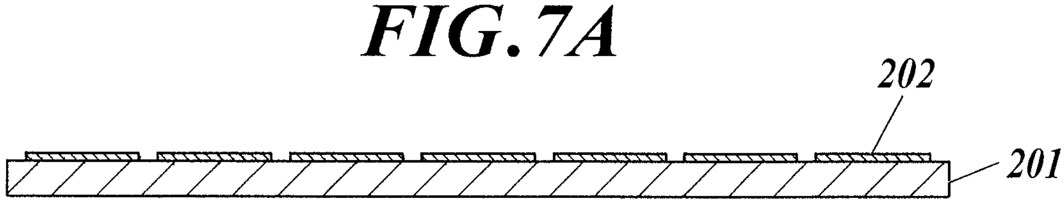


FIG. 7B

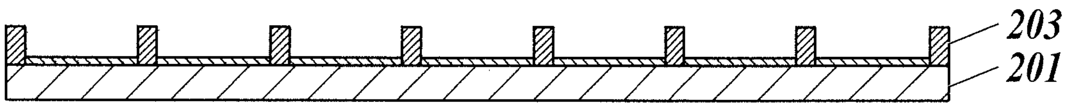


FIG. 7C

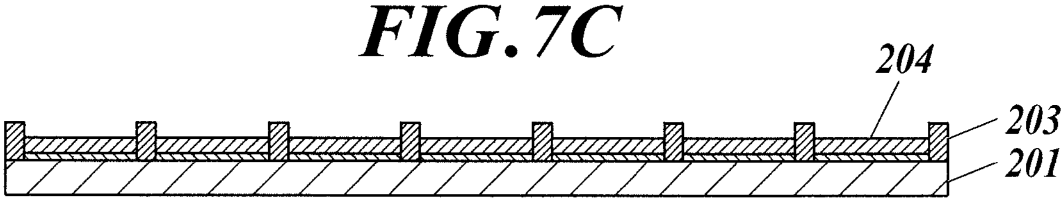


FIG. 7D

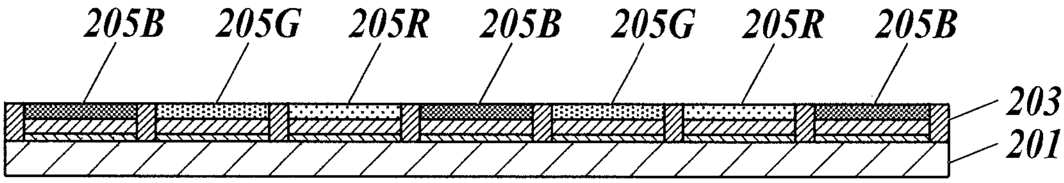


FIG. 7E

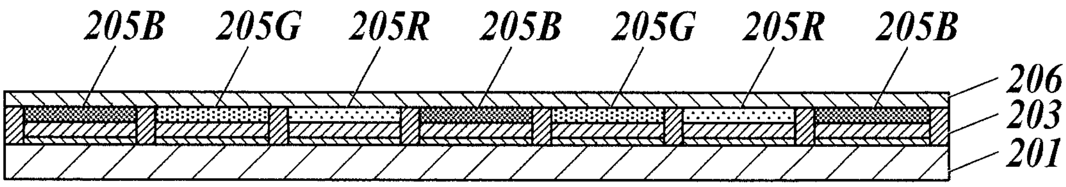

## INTERNATIONAL SEARCH REPORT

International application No.
PCT/JP2014/058808

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
H01L51/50, C09K11/06, G09F9/30

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2007-294720 A (Konica Minolta Holdings, Inc.), 08 November 2007 (08.11.2007), paragraphs [0065] to [0067], [0078], [0080], [0192] (Family: none) | 1,3,5-9 |
| X | JP 2010-126571 A (Toyo Ink Manufacturing Co., Ltd.), 10 June 2010 (10.06.2010), claim 1; paragraphs [0085] to [0106], [0151] to [0153], [0167], [0187] to [0188] (Family: none) | 1,3,5-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 June, 2014 (24.06.14) | 01 July, 2014 (01.07.14) |
| Name and mailing address of the ISA/ Japanese Patent Office | Authorized officer |
| Facsimile No. | Telephone No. |

Form PCT/ISA/210 (second sheet) (July 2009)

INTERNATIONAL SEARCH REPORT

International application No. PCT/JP2014/058808

C (Continuation). DOCUMENTS CONSIDERED TO BE RELEVANT

| Category* | Citation of document, with indication, where appropriate, of the relevant passages | Relevant to claim No. |
|---|---|---|
| X | JP 2009-173565 A (Toyo Ink Manufacturing Co., Ltd.), 06 August 2009 (06.08.2009), claim 1; paragraphs [0003], [0048] to [0062], [0115] to [0117], [0170] to [0171] (Family: none) | 1,3,5-8 |
| X | JP 2008-255324 A (Toyo Ink Manufacturing Co., Ltd.), 23 October 2008 (23.10.2008), claims; paragraphs [0041] to [0044], [0068], [0083], [0138] to [0139] (Family: none) | 1-6,8-9 |
| X | JP 2004-155665 A (Mitsubishi Chemical Corp.), 03 June 2004 (03.06.2004), paragraphs [0075] to [0076], [0081], [0087] (Family: none) | 1-5,8 |
| X | WO 2012/109747 A1 (UNIVERSITY OF WATERLOO), 23 August 2012 (23.08.2012), paragraphs [0076], [0081], [0106]; fig. 5 to 6 (Family: none) | 1-5 |

Form PCT/ISA/210 (continuation of second sheet) (July 2009)

**INTERNATIONAL SEARCH REPORT**

International application No.
PCT/JP2014/058808

Continuation of A. CLASSIFICATION OF SUBJECT MATTER
(International Patent Classification (IPC))

*H01L51/50*(2006.01)i, *C07D403/14*(2006.01)i, *C07D405/14*(2006.01)i, *C07D413/04*(2006.01)i, *C07D413/14*(2006.01)i, *C07D471/14*(2006.01)i, *C07D487/04*(2006.01)i, *C07D487/14*(2006.01)i, *C07D491/048*(2006.01)i, *C07D491/147*(2006.01)i, *C07D495/14*(2006.01)i, *C07D498/14*(2006.01)i, *C07D513/14*(2006.01)i, *C07D513/16*(2006.01)i, *C09K11/06*(2006.01)i, *G09F9/30*(2006.01)i, *H01L27/32*(2006.01)i

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007243101 A
- JP 2012051667 A
- JP 2007023101 A
- JP 5076891 B
- JP 2011134013 A
- US 7420203 B
- US 7473923 B
- US 6872472 B
- US 6107734 A
- US 6337492 B
- JP 2011096679 A
- JP 2010192719 A
- JP 2009076929 A
- JP 2008078414 A
- JP 2007059848 A
- JP 2005094130 A
- JP 2011156793 A
- JP 2011213643 A
- JP 2010093181 A
- WO 2009100991 A
- US 2006835469 A
- US 20060202194 A
- US 20070087321 A
- WO 2009050290 A
- WO 2009000673 A
- US 20090108737 A
- US 20090039776 A
- US 20090165846 A
- US 20080015355 A
- US 20060263635 A
- US 7090928 B
- WO 2006009024 A
- WO 2006056418 A
- WO 2005019373 A
- WO 2005123873 A
- US 20060251923 A
- US 20050260441 A
- US 20070190359 A
- US 20080297033 A
- US 2006103874 A
- WO 2010032663 A
- WO 2008140115 A
- WO 2011134013 A
- WO 2011157339 A
- WO 2010086089 A
- WO 2009113646 A
- WO 2012020327 A
- WO 2011051404 A
- WO 2011073149 A
- JP 2012069737 A
- JP 2011181303 A
- JP 2009114086 A
- JP 2003081988 A
- JP 2002302671 A
- JP 2002363552 A
- JP 2002203683 A
- JP 2002363227 A
- JP 2002234888 A
- JP 2002280183 A
- JP 2002299060 A
- JP 2002302516 A
- JP 2002305083 A
- JP 2002305084 A
- US 20090017330 A
- US 20090030202 A
- US 20050238919 A
- WO 2001039234 A
- WO 2009021126 A
- WO 2008056746 A
- WO 2007063796 A
- WO 2007063754 A
- WO 2004107822 A
- WO 2006114966 A
- WO 2009086028 A
- WO 2009003898 A
- WO 2012023947 A
- JP 2008074939 A
- JP 2007254297 A
- EP 2034538 A
- JP 4297076 A
- JP 10270172 A
- JP 2000196140 A
- JP 2001102175 A
- US 20090115316 A
- US 20090179554 A
- WO 2003060956 A
- WO 2008132085 A
- US 2009030202 A
- US 2004080975 A
- US 2005085387 A
- US 2006067931 A
- US 2007086552 A
- US 2008114690 A
- US 2009069442 A
- US 2009066779 A
- US 2009054253 A
- US 2011086935 A
- US 2010150593 A

- US 2010047707 A
- JP 2010251675 A
- JP 2009209133 A
- JP 2009124114 A
- JP 2008277810 A
- JP 2006156445 A
- JP 2003031367 A
- WO 2012115034 A
- JP 6325871 A
- JP 9017574 A
- JP 10074586 A
- JP 2003519432 W
- JP 2006135145 A
- JP 11251067 A
- US 20030162053 A
- US 20060240279 A
- US 20080220265 A
- WO 2007002683 A
- WO 2009018009 A
- EP 650955 A
- US 20080124572 A
- US 20070278938 A
- US 20080106190 A
- US 20080018221 A
- JP 9045479 A
- JP 9260062 A
- JP 8288069 A

**Non-patent literature cited in the description**

- Shinpen Shikisai Kagaku Handobukku. University of Tokyo Press, 1985, 108
- **BUNKO II.** Dai 4 Han Jikken Kagaku Koza 7. Maruzen Co, 1992, vol. 7, 398
- *Nature,* 1998, vol. 395, 151
- *Adv. Mater.,* 2007, vol. 19, 739
- *Adv. Mater.,* 2005, vol. 17, 1059
- *Inorg. Chem.,* 2001, vol. 40, 1704
- *Chem. Mater.,* 2004, vol. 16, 2480
- *Adv. Mater.,* 2004, vol. 16, 2003
- *Appl. Phys. Lett.,* 2005, vol. 86, 153505
- *Inorg. Chem.,* 2003, vol. 42, 1248
- *Angew. Chem. Int. Ed.,* 2008, vol. 47, 1
- *Chem. Mater.,* 2006, vol. 18, 5119
- *Inorg. Chem.,* 2007, vol. 46, 4308
- *Appl. Phys. Lett.,* 1999, vol. 74, 1361
- *J. Appl. Phys.,* 2004, vol. 95, 5773
- *Appl. Phys. Lett.,* 1999, vol. 75, 4
- *Appl. Phys. Lett.,* 2002, vol. 81, 162
- *Appl. Phys. Lett.,* 2001, vol. 79, 156
- Yuki EL Soshi To Sono Kogyoka Saizensen. Denky-oku Zairyo. N.T.S Co., Ltd, 30 November 1998, 123-166
- **J. HUANG.** *Appl. Phys. Lett.,* 2002, vol. 80, 13-9
- *Appl. Phys. Lett.,* 1996, vol. 69, 2160
- *Appl. Phys. Lett.,* 2001, vol. 78, 673
- *Appl. Phys. Lett.,* 2007, vol. 90, 183503
- *Appl. Phys. Lett.,* 1987, vol. 51, 913
- *Synth. Met.,* 1997, vol. 87, 171
- *Synth. Met.,* 1997, vol. 91, 209
- *Synth. Met.,* 2000, vol. 111, 421
- *SID Symposium Digest,* 2006, vol. 37, 923